(19) Europäisches Patentamt
European Patent Office
Office européen des brevets

(11) Veröffentlichungsnummer: **0 254 955 B1**

(12) # EUROPÄISCHE PATENTSCHRIFT

(45) Veröffentlichungstag der Patentschrift: **27.05.92**

(51) Int. Cl.$^5$: **C07D 471/04**, A61K 31/55, //(C07D471/04,243:00,221:00)

(21) Anmeldenummer: **87110190.3**

(22) Anmeldetag: **15.07.87**

(54) **Substituierte Pyrido-[2,3-b][1,4]benzodiazepin-6-one, Verfahren zu ihrer Herstellung und diese Verbindungen enthaltende Arzneimittel.**

(30) Priorität: **31.07.86 DE 3626095**

(43) Veröffentlichungstag der Anmeldung:
**03.02.88 Patentblatt 88/05**

(45) Bekanntmachung des Hinweises auf die Patenterteilung:
**27.05.92 Patentblatt 92/22**

(84) Benannte Vertragsstaaten:
**AT BE CH DE ES FR GB GR IT LI LU NL SE**

(56) Entgegenhaltungen:
**EP-A- 0 156 191**
**US-A- 3 660 380**
**US-A- 4 210 648**

(73) Patentinhaber: **Dr. Karl Thomae GmbH**
**Postfach 1755**
**W-7950 Biberach (Riss)(DE)**

(72) Erfinder: **Engel, Wolfgang, Dr. Dipl.-Chem.**
**Mozartstrasse 13**
**W-7950 Biberach 1(DE)**
Erfinder: **Eberlein, Wolfgang, Dr. Dipl.-Chem.**
**Obere Au 6**
**W-7950 Biberach 1(DE)**
Erfinder: **Trummlitz, Günter, Dr. Dipl.-Chem.**
**Buchenweg 27**
**W-7951 Warthausen(DE)**
Erfinder: **Mihm, Gerhard, Dr. Dipl.-Chem.**
**Nickeleshalde 5/1**
**W-7950 Biberach 1(DE)**
Erfinder: **Mayer, Nobert, Dr.**
**Friedr.-Ebert-Str. 66**
**W-7950 Biberach 1(DE)**
Erfinder: **de Jonge, Adriaan, Dr.**
**de Boomgaard 19**
**NL-3971 LD Driebergen(NL)**

EP 0 254 955 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft substituierte 6H-Pyrido-[2,3-b][l,4]benzodiazepin-6-one, Verfahren zu ihrer Herstellung und diese Verbindungen enthaltende Arzneimittel.

Aus EP-A-0 039 5l9 und 0 057 428 sowie aus US-A 3.660.380; 3.69l.l59; 4.2l3.984; 4.2l3.985; 4.2l0.648, 4.4l0.527; 4.424.225; 4.424.222 und 4.424.226 sind bereits kondensierte Diazepinone mit ulcushemmenden und magensaftsekretionshemmenden Eigenschaften bekannt.

In EP-A-0 l56 l9l (US-Patent 4 550 l07) ist beschrieben, daß durch Einführung neuartiger Aminoacylreste gegenüber den Verbindungen der obengenannten Publikationen völlig andersartige, wertvolle pharmakologische Eigenschaften induziert werden können. Gegenüber diesen Verbindungen zeichnen sich die erfindungsgemäßen, substituierten Pyrido[2,3-b][l,4]benzodiazepin-6-one überraschenderweise bei vergleichbarer oder verbesserter Selektivität durch eine wesentlich verstärkte Wirkung und Resorption nach oraler Gabe aus.

Die substituierten Pyrido[2,3-b][l,4]benzodiazepin-6-one besitzen die allgemeine Formel I

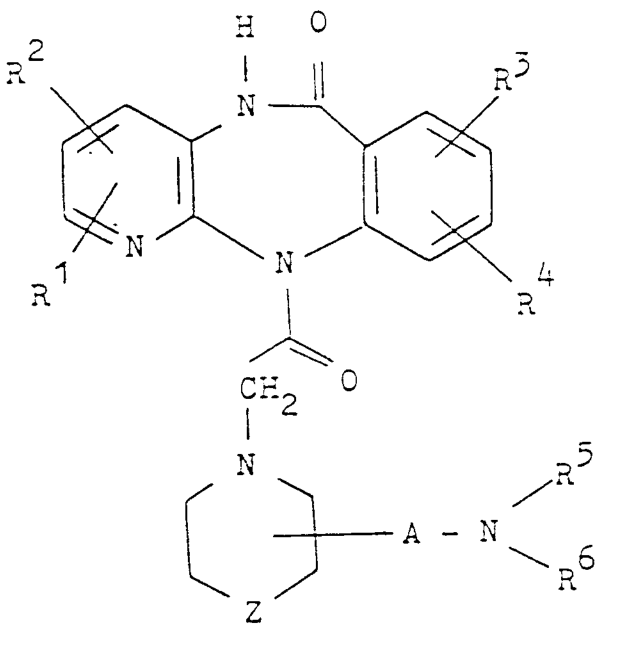

(I),

in der

$R^1$ eine Alkylgruppe mit l bis 4 C-Atomen, ein Chloratom oder ein Wasserstoffatom;

$R^2$ ein Wasserstoffatom oder eine Methylgruppe;

$R^3$ und $R^4$ jeweils ein Wasserstoffatom, ein Fluor-, Chlor- oder Bromatom oder eine Alkylgruppe mit l bis 4 C-Atomen bedeuten, jedoch mit der Maßgabe, daß wenigstens einer der Reste $R^1$, $R^2$, $R^3$ und $R^4$ von Wasserstoff verschieden ist,

und in der

$R^5$ und $R^6$ gleiche oder voneinander verschiedene Alkylreste mit bis zu 6 Kohlenstoffatomen sind oder zusammen mit dem dazwischenliegenden Stickstoffatom einen 4- bis 7-gliedrigen, gesättigten, monocyclischen, heteroaliphatischen Ring bilden, der gegebenenfalls durch ein Sauerstoffatom oder durch die $N\text{-}CH_3$-Gruppe unterbrochen sein kann,

und in der

Z entweder eine Einfachbindung oder ein Sauerstoffatom, eine Methylen- oder l,2-Ethylengruppe

und

A eine Methylengruppe in 2- oder 3-Stellung des heteroaliphatischen Rings, bei Verknüpfung in 3-Stellung auch eine Einfachbindung ist.

Bevorzugt werden solche Verbindungen der allgemeinen Formel I, die als Aminoacylrest den [[2-[(Diethylamino)methyl]-l-piperidinyl]-acetyl]-Rest tragen und in 2-, 8- oder 9-Stellung monochlor- oder monomethylsubstituiert oder in 8- oder 9-Stellung monobrom- oder monoethylsubstituiert sind.

Die Verbindungen der allgemeinen Formel I können nach Umsetzung mit anorganischen oder organischen Säuren auch in Form ihrer physiologisch verträglichen Salze vorliegen. Als Säuren haben sich beispielsweise Salzsäure, Bromwasserstoffsäure, Schwefelsäure, Methylschwefelsäure, Phosphorsäure, Weinsäure, Fumarsäure, Zitronensäure, Maleinsäure, Bernsteinsäure, Gluconsäure, Äpfelsäure, p-Toluolsulfonsäure, Methansulfonsäure oder Amidosulfonsäure als geeignet erwiesen.

Zur Erläuterung des Erfindungsgegenstandes seien als Beispiele folgende Verbindungen genannt:

2-Chlor-ll-[[2-[(diethylamino)methyl]-l-piperidinyl]acetyl]-5,ll-dihydro-6H-pyrido[2,3-b][l,4]benzodiazepin-6-on
9-Chlor-ll-[[2-[(diethylamino)methyl]-l-piperidinyl]acetyl]-5,ll-dihydro-6H-pyrido[2,3-b][l,4]benzodiazepin-6-on
ll-[[2-[(Diethylamino)methyl]-l-piperidinyl]acetyl]-5,ll-dihydro-2-methyl-6H-pyrido[2,3-b][l,4]benzodiazepin-6-on
ll-[[2-[(Diethylamino)methyl]-l-piperidinyl]acetyl]-5,ll-dihydro-8-methyl-6H-pyrido[2,3-b][l,4]benzodiazepin-6-on
ll-[[2-[(Diethylamino)methyl]-l-piperidinyl]acetyl]-5,ll-dihydro-9-methyl-6H-pyrido[2,3-b][l,4]benzodiazepin-6-on
8-Chlor-ll-[[2-[(diethylamino)methyl]-l-piperidinyl]acetyl]-5,ll-dihydro-6H-pyrido[2,3-b][l,4]benzodiazepin-6-on
ll-[[2-[(Diethylamino)methyl]-l-piperidinyl]acetyl]-5,ll-dihydro-2,4,l0-trimethyl-6H-pyrido[2,3-b][l,4]-benzodiazepin-6-on
ll-[[2-[(Diethylamino)methyl]-l-piperidinyl]acetyl]-5,ll-dihydro-2,4,8-trimethyl-6H-pyrido[2,3-b][l,4]-benzodiazepin-6-on
ll-[[2-[(Diethylamino)methyl]-l-piperidinyl]acetyl]-5,ll-dihydro-2,8-dimethyl-6H-pyrido[2,3-b][l,4]benzodiazepin-6-on
8,l0-Dichlor-ll-[[2-[(diethylamino)methyl]-l-piperidinyl]-acetyl]-5,ll-dihydro-6H-pyrido[2,3-b][l,4]benzodiazepin-6-on
ll-[[2-[(Diethylamino)methyl]-l-piperidinyl]acetyl]-5,ll-dihydro-2,l0-dimethyl-6H-pyrido[2,3-b][l,4]-benzodiazepin-6-on
ll-[[2-[(Diethylamino)methyl]-l-piperidinyl]acetyl]-5,ll-dihydro-2,4-dimethyl-6H-pyrido[2,3-b][l,4]benzodiazepin-6-on
2,9-Dichlor-ll-[[2-[(diethylamino)methyl]-l-piperidinyl]-acetyl]-5,ll-dihydro-6H-pyrido[2,3-b][l,4]benzodiazepin-6-on
2,l0-Dichlor-ll-[[2-[(diethylamino)methyl]-l-piperidinyl]acetyl]-5,ll-dihydro-6H-pyrido[2,3-b][l,4]benzodiazepin-6-on
ll-[[2-[(Diethylamino)methyl]-l-piperidinyl]acetyl]-5,ll-dihydro-l0-methyl-6H-pyrido[2,3-b][l,4]benzodiazepin-6-on
l0-Brom-ll-[[2-[(diethylamino)methyl]-l-piperidinyl]acetyl]-5,ll-dihydro-6H-pyrido[2,3-b][l,4]benzodiazepin-6-on
ll-[[2-[(Diethylamino)methyl]-l-piperidinyl]acetyl]-5,ll-dihydro-8-fluor-6H-pyrido[2,3-b][l,4]benzodiazepin-6-on
ll-[[2-[(Diethylamino)methyl]-l-piperidinyl]acetyl]-5,ll-dihydro-9-fluor-6H-pyrido[2,3-b][l,4]benzodiazepin-6-on
ll-[[2-[(Diethylamino)methyl]-l-piperidinyl]acetyl]-5,ll-dihydro-7-methyl-6H-pyrido[2,3-b][l,4]benzodiazepin-6-on
ll-[[2-[(Diethylamino)methyl]-l-piperidinyl]acetyl]-5,ll-dihydro-7-fluor-6H-pyrido[2,3-b][l,4]benzodiazepin-6-on
8-Brom-ll-[[2-[(diethylamino)methyl]-l-piperidinyl]acetyl]-5,ll-dihydro-6H-pyrido[2,3-b][l,4]benzodiazepin-6-on
ll-[[2-[(Diethylamino)methyl]-l-piperidinyl]acetyl]-5,ll-dihydro-8-ethyl-6H-pyrido[2,3-b][l,4]benzodiazepin-6-on
ll-[[2-[(Diethylamino)methyl]-l-piperidinyl]acetyl]-5,ll-dihydro-8,9-dimethyl-6H-pyrido[2,3-b][l,4]benzodiazepin-6-on
(+)-9-Chlor-ll-[[2-[(diethylamino)methyl]-l-piperidinyl]acetyl]-5,ll-dihydro-6H-pyrido[2,3-b][l,4]benzodiazepin-6-on
(-)-9-Chlor-ll-[[2-[(diethylamino)methyl]-l-piperidinyl]acetyl]-5,ll-dihydro-6H-pyrido[2,3-b][l,4]benzodiazepin-6-on
9-Chlor-5,ll-dihydro-ll-[[2-[(dimethylamino)methyl]-l-piperidinyl]acetyl]-6H-pyrido[2,3-b][l,4]benzodiazepin-6-on
9-Chlor-5,ll-dihydro-ll-[[2-[(l-pyrrolidinyl)methyl]-l-piperidinyl]acetyl]-6H-pyrido[2,3-b][l,4]benzodiazepin-6-on
9-Chlor-5,ll-dihydro-ll-[[2-[(4-morpholinyl)methyl]-l-piperidinyl]acetyl]-6H-pyrido[2,3-b][l,4]benzodiazepin-6-on
9-Chlor-5,ll-dihydro-ll-[[2-[(4-methyl-l-piperazinyl)methyl]-l-piperidinyl]acetyl]-6H-pyrido[2,3-b][l,4]-benzodiazepin-6-on
9-Chlor-ll-[[3-[(diethylamino)methyl]-l-piperidinyl]acetyl]-5,ll-dihydro-6H-pyrido[2,3-b][l,4]benzodiazepin-6-on
(S)-9-Chlor-ll-[[2-[(diethylamino)methyl]-l-pyrrolidinyl]acetyl]-5,ll-dihydro-6H-pyrido[2,3-b][l,4]benzodiazepin-6-on
9-Chlor-5,ll-dihydro-ll-[[3-(dimethylamino)-l-piperidinyl]acetyl]-6H-pyrido[2,3-b][l,4]benzodiazepin-6-on
D,L-9-Chlor-ll-[[3-[(diethylamino)methyl]-4-morpholinyl]acetyl]-5,ll-dihydro-6H-pyrido[2,3-b][l,4]-

benzodiazepin-6-on

9-Chlor-5,ll-dihydro-ll-[[2-[(ethylmethylamino)methyl]hexahydro-lH-azepin-l-yl]acetyl]-6H-pyrido[2,3-b][l,4]-benzodiazepin-6-on

5,ll-Dihydro-9-methyl-ll-[[2-[(propylmethylamino)methyl]hexahydro-lH-azepin-l-yl]acetyl]-6H-pyrido[2,3-b][l,4]-benzodiazepin-6-on

5,ll-Dihydro-9-methyl-ll-[[2-[[methyl-(l-methylethyl)amino]methyl]-l-piperidinyl]acetyl]-6H-pyrido[2,3-b][l,4]-benzodiazepin-6-on

Erfindungsgemäß erhält man die basisch substituierten kondensierten Diazepinone der allgemeinen Formel I nach folgenden Verfahren:

a) Durch Umsetzung von Halogenacylverbindungen der allgemeinen Formel II

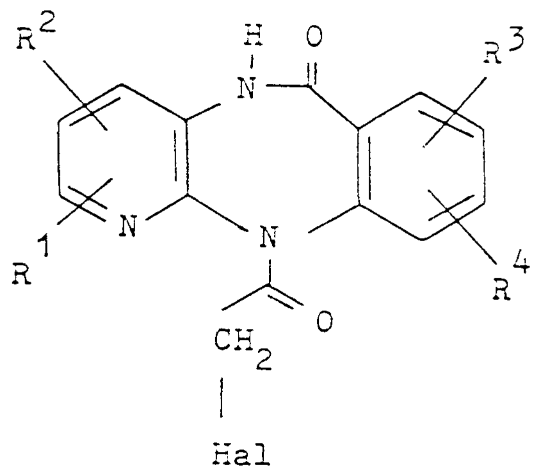

(II),

in der $R^1$, $R^2$, $R^3$ und $R^4$ die angegebenen Bedeutungen haben und Hal ein Chlor-, Brom- oder lodatom ist, mit sekundären Aminen der allgemeinen Formel III,

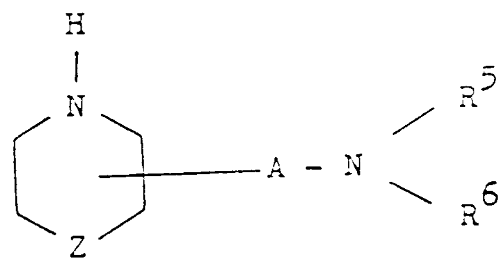

(III),

in der $R^5$, $R^6$, A und Z die eingangs definierten Bedeutungen besitzen.

Die Aminierung erfolgt in einem inerten Lösungsmittel bei Temperaturen zwischen -l0 ° C und der Siedetemperatur des Lösungsmittels, vorzugsweise entweder mit wenigstens 2 Molen sekundärem Amin der allgemeinen Formel III oder mit l bis 2 Molen eines sekundären Amins der allgemeinen Formel III und einer Hilfsbase. Als Lösungsmittel kommen beispielsweise chlorierte Kohlenwasserstoffe, wie Methylenchlorid, Chloroform oder Dichlorethan; offenkettige oder cyclische Ether, wie Diethylether, Tetrahydrofuran oder Dioxan; aromatische Kohlenwasserstoffe, wie Benzol, Toluol, Xylol, Chlorbenzol oder Pyridin; Alkohole, wie Ethanol oder Isopropanol; Ketone, wie Aceton; Acetonitril, Dimethylformamid oder l,3-Dimethyl-2-imidazolidinon in Frage. Als Hilfsbasen seien beispielsweise tertiäre organische Basen, wie Triethylamin, N-Methylpiperidin, Diethylanilin, Pyridin und 4-(Dimethylamino)pyridin oder anorganische Basen, wie Alkalimetall-oder Erdalkalimetallcarbonate oder -hydrogencarbonate, -hydroxide oder -oxide genannt. Gegebenenfalls kann die Reaktion durch Zusatz von Alkalimetalliodiden beschleunigt werden. Die Reaktionszeiten betragen je nach Menge und Art des eingesetzten Amins der allgemeinen Formel III zwischen l5 Minuten und 80 Stunden.

b.) Durch Acylierung von substituierten Pyrido[2,3-b][l,4]benzodiazepin-6-onen der allgemeinen Formel

IV,

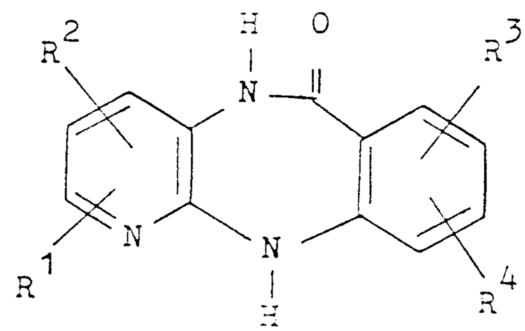

(IV),

worin $R^1$, $R^2$, $R^3$ und $R^4$ die oben angegebenen Bedeutungen haben, mit Carbonsäurederivaten der allgemeinen Formel V,

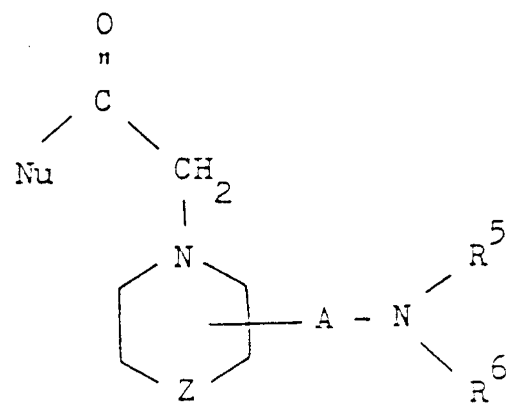

(V),

worin $R^5$, $R^6$, A und Z die oben angegebenen Bedeutungen haben und Nu eine nucleofuge Gruppe bzw. Abgangsgruppe darstellt.

Die Umsetzung der Verbindungen der allgemeinen Formel IV mit den Säurederivaten der allgemeinen Formel V erfolgt in an sich bekannter Weise. Die Abgangsgruppe Nu ist eine Gruppe, die gemeinsam mit der Carbonylgruppe, an die sie gebunden ist, ein reaktives Carbonsäurederivat bildet. Als reaktive Carbonsäurederivate seien beispielsweise Säurehalogenide, -ester, -anhydride oder gemischte Anhydride, wie sie aus Salzen der entsprechenden Säuren (Nu = OH) und Säurechloriden, wie Phosphoroxidchlorid, Diphosphorsäuretetrachlorid oder Chlorameisensäureestern gebildet werden oder die bei der Umsetzung von Verbindungen der allgemeinen Formel V (Nu = OH) mit N-Alkyl-2-halogenpyridiniumsalzen entstehenden N-Alkyl-2-acyloxypyridiniumsalze genannt.

Bevorzugt wird die Reaktion mit den gemischten Anhydriden starker Mineralsäuren, insbesondere der Dichlorphosphorsäure, durchgeführt. Die Reaktion wird gegebenenfalls in Gegenwart eines säurebindenden Mittels (Protonenakzeptors) durchgeführt. Als geeignete Protonenakzeptoren seien beispielsweise Alkalimetallcarbonate oder -hydrogencarbonate, wie Natriumcarbonat oder Kaliumhydrogencarbonat; tertiäre organische Amine, wie Pyridin, Triethylamin, Ethyldiisopropylamin, 4-(Dimethylamino)pyridin, oder Natriumhydrid genannt. Die Reaktion wird bei Temperaturen zwischen -25 C und l30 ° C in einem inerten Lösungsmittel durchgeführt. Als inerte Lösungsmittel kommen beispielsweise chlorierte aliphatische Kohlenwasserstoffe, wie Methylenchlorid, l,2-Dichlorethan; offenkettige oder cyclische Ether, wie Diethylether, Tetrahydrofuran oder l,4-Dioxan; aromatische Kohlenwasserstoffe, wie Benzol, Toluol, Xylol, o-Dichlorbenzol; polare aprotische Lösungsmittel wie Acetonitril, Dimethylformamid oder Hexamethylphosphorsäuretriamid; oder Gemische davon in Frage. Die Reaktionszeiten betragen je nach Menge und Art des eingesetzten Acylierungsmittels der allgemeinen Formel V zwischen l5 Minuten und 80 Stunden. Es ist nicht nötig, die Verbindungen der allgemeinen Formel V in reiner Form herzustellen, sie können vielmehr im Reaktionsansatz in bekannter Weise in situ erzeugt werden.

So erhaltene Basen der allgemeinen Formel I können anschließend in ihre Säureadditionssalze oder

erhaltene Säureadditionssalze in die freien Basen oder andere pharmakologisch verträgliche Säureadditionssalze übergeführt werden.

Die erfindungsgemäßen basisch substituierten kondensierten 6H-Pyrido[2,3-b][I,4]benzodiazepin-6-one der allgemeinen Formel I enthalten bis zu zwei unabhängige chirale Elemente, davon ein asymmetrisches Kohlenstoffatom in der Seitenkette. Als zweites chirales Element ist der acylierte Tricyclus selbst anzusehen, der in zwei spiegelbildlichen Formen vorliegen kann. Von der Natur des Tricyclus hängt es ab, ob die Energiebarriere für eine Inversion an diesem Zentrum so hoch ist, daß die einzelnen Isomeren bei Zimmertemperatur stabil sind und isolierbar werden. Es hat sich gezeigt, daß bei Verbindungen der allgemeinen Formel I, die in 4-, 7- und I0-Stellung unsubstituiert sind, die erforderliche Aktivierungsenergie so stark vermindert ist, daß Diastereomere bei Zimmertemperatur nicht mehr nachgewiesen geschweige denn präparativ isoliert werden können.

Die erfindungsgemäßen aminoacylierten kondensierten Diazepinone der allgemeinen Formel I enthalten also in der Regel 2 chirale Zentren, von denen eines bei Zimmertemperatur unter Umständen nicht konfigurationsstabil ist. Diese Verbindungen können deshalb in zwei diastereomeren oder jeweils als enantiomere (+)- und (-)-Formen auftreten. Die Erfindung umfaßt die einzelnen Isomeren ebenso wie ihre Gemische. Die Trennung der jeweiligen Diastereomeren gelingt auf Grund der unterschiedlichen physikochemischen Eigenschaften, z.B. durch fraktioniertes Umkristallisieren aus geeigneten Lösungsmitteln, durch Hochdruckflüssigkeitschromatographie, Säulenchromatographie oder gaschromatographische Verfahren.

Die Spaltung evtl. Razemate der Verbindungen der allgemeinen Formel I kann nach bekannten Verfahren durchgeführt werden, beispielsweise unter Verwendung einer optisch aktiven Säure, wie (+)- oder (-)-Weinsäure, oder eines Derivats davon, wie (+)- oder (-)-Diacetylweinsäure, (+)- oder (-)-Monomethyltartrat oder (+)-Camphersulfonsäure.

Nach einem üblichen Verfahren zur Isomerentrennung wird das Racemat einer Verbindung der allgemeinen Formel I mit einer der vorstehend angegebenen optisch aktiven Säuren in äquimolarer Menge in einem Lösungsmittel umgesetzt und die erhaltenen kristallinen diastereomeren Salze werden unter Ausnutzung ihrer verschiedenen Löslichkeit getrennt. Diese Umsetzung kann in jeder Art von Lösungsmittel durchgeführt werden, solange dieses einen ausreichenden Unterschied in der Löslichkeit der Salze aufweist. Vorzugsweise werden Methanol, Ethanol oder deren Gemische, beispielsweise im Volumenverhältnis 50:50, verwendet. Sodann wird jedes der diastereomeren Salze in Wasser gelöst, mit einer Base, wie Natriumcarbonat oder Kaliumcarbonat, neutralisiert und dadurch die entsprechende freie Verbindung in der (+)- oder (-)-Form erhalten.

Jeweils nur ein Enantiomeres bzw. ein Gemisch zweier optisch aktiver unter die allgemeine Formel I fallender diastereomerer Verbindungen wird auch dadurch erhalten, daß man die oben beschriebenen Synthesen mit nur einem Enantiomeren der allgemeinen Formel III bzw. V durchführt.

Zur Herstellung der Halogenacylverbindungen der allgemeinen Formel II werden die Ausgangsverbindungen der allgemeinen Formel IV mit Verbindungen der allgemeinen Formel Hal-$CH_2$CO-Hal′ (VII) oder [Hal-$CH_2$-CO]$_2$0 (VIII), worin Hal′ eine der Bedeutungen von Hal hat und Hal wie oben definiert ist, umgesetzt. Diese Acylierung wird ohne oder vorzugsweise in einem inerten Lösungsmittel bei Raumtemperatur oder erhöhter Temperatur, maximal bei der Siedetemperatur des Lösungsmittels, gegebenenfalls in Gegenwart einer Hilfsbase und/oder eines Acylierungskatalysators, vorgenommen. Die Säurehalogenide der allgemeinen Formel VII sind gegenüber den Säureanhydriden der allgemeinen Formel VIII bevorzugt. Als Säurehalogenid der allgemeinen Formel VII ist Chloracetylchlorid, als Säureanhydrid der allgemeinen Formel VIII ist Chloressigsäureanhydrid bevorzugt. Als Lösungsmittel seien beispielsweise genannt aromatische Kohlenwasserstoffe, wie Toluol, Xylol oder Chlorbenzol; offenkettige oder cyclische Ether, wie Diisopropylether oder Dioxan; chlorierte Kohlenwasserstoffe, wie Dichlorethan, andere Lösungsmittel, wie Pyridin, Acetonitril oder Dimethylformamid.

Als Hilfsbasen seien z.B. tertiäre organische Basen, wie Triethylamin und Ethyldiisopropylamin, oder Pyridin; oder anorganische Basen, wie wasserfreie Alkalimetall- oder Erdalkalimetallcarbonate oder -hydrogencarbonate oder Erdalkalimetalloxide genannt. Als Acylierungskatalysatoren kommen beispielsweise in Frage Imidazol, Pyridin oder 4-Dimethylaminopyridin.

Bedeutet in einer Verbindung der allgemeinen Formel II Hal ein Chloratom, so kann es durch Umsetzung mit Natriumiodid in Aceton oder Ethanol leicht gegen das reaktivere Iod ausgetauscht werden.

Ausgangsverbindungen der allgemeinen Formel III, in der Z eine Methylengruppe ist und $R^5$, $R^6$ und A die eingangs definierten Bedeutungen haben, sind bekannt oder lassen sich in Analogie zu bekannten Verfahren herstellen. So erhält man beispielsweise solche Verbindungen der allgemeinen Formel III, in der A eine Methylengruppen ist, durch Umsetzung von 2- oder 3-(Chlormethyl)pyridin-hydrochlorid mit einem sekundären Amin der allgemeinen Formel VI,

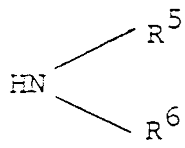

(VI)

in der $R^5$ und $R^6$ wie eingangs definiert sind (in Analogie zu A. Fischer u.a., Can. J. Chem. 56, 3059-3067 [1967]) und anschließende katalytische Hydrierung des erhaltenen tertiären Picolylamins, beispielsweise in ethanolisch-salzsaurer Lösung und unter Verwendung von Platin(IV)-oxid als Katalysator (siehe auch: F.F. Blicke u.a., J. Org. Chemistry 26, 3258[1961]) oder in Eisessig in Gegenwart von Platin(IV)-oxid (siehe auch: W.F. Minor u.a., J. Med. Pharm. Chem. 5, 96, 105ff [1962] und A.H. Sommers u.a., J. Amer. chem. Soc., 75, 57, 58ff. [1953]).

Die unter die Diamine der allgemeinen Formel III fallenden 2-[(Dialkylamino)methyl]pyrrolidine, bei denen Z eine Einfachbindung bedeutet, lassen sich nach oder in Analogie zu T. Sone u.a., Chem. Pharm. Bull. (Tokyo) 21, 2331 [1973] durch Reduktion entsprechender Prolinamide mit Lithiumaluminiumhydrid erhalten. Ersetzt man in dieser Synthese Prolin durch Hexahydro-1H-azepin-2-carbonsäure (siehe auch H.T. Nagasawa u.a., J. Med. Chem. 14, 501 [1971]), so gelangt man zu den unter die allgemeine Formel III fallenden 2-substituierten Hexahydro-1H-azepinen, in denen Z eine Ethylengruppe und A eine Methylengruppe darstellen und $R^5$ und $R^6$ die eingangs angegebenen Bedeutungen haben

Die unter die allgemeine Formel III fallenden 3-[(Dialkylamino)methyl]piperidine lassen sich auch aus entsprechenen Nicotinsäureamiden nach V.M. Mićović u.a., J. org. Chem. 18, 1196 [1953] bzw. F. Haglid u.a., Acta. Chem. Scand. 17, 1741 [1963] herstellen.

3-(Dialkylamino)-pyrrolidine, -piperidine und -hexahydro-1H-azepine (III: $R^5$ und $R^6$ wie eingangs angegeben; Z eine Methylen- oder Ethylengruppe oder eine Einfachbindung; A eine Einfachbindung) lassen sich nach den Angaben von H.R. Bürki u.a., Eur. J.Med.Chem. 13, 479-485 [1978] und Smithkline Corp., US-A 3.980.788; C.A. 85, P 182415z [1976] aus N-Benzyl-3-pyrrolidinon, -3-piperidinon bzw. -hexahydro-1H-azepin-3-on oder in Analogie dazu herstellen.

3-[(Dialkylamino)methyl]-pyrrolidine, bei welchen in der Formel III $R^5$ und $R^6$ wie oben angegeben definiert sind, Z eine Einfachbindung und A eine Methylengruppe in 3-Stellung darstellen, erhält man aus der leicht zugänglichen 1-Benzyl-2,3-dihydro-5(4H)-oxo-1H-pyrrol-3-carbonsäure (Yao-Hua Wu und R.F. Feldkamp, J. Org. Chem. 26, 1519 [1961], durch aufeinanderfolgende Umsetzung mit Thionylchlorid, einem Amin der allgemeinen Formel VI und nachfolgende Reduktion mit Lithiumaluminiumhydrid, sowie hydrogenolytische Entfernung des Benzylrestes. Analog kann man auch 3-[(Dialkylamino)methyl]-hexahydro-1H-azepine der Formel III, worin Z eine 1,2-Ethylengruppe darstellt, synthetisieren.

Verbindungen der allgemeinen Formel III, in der Z ein Sauerstoffatom ist und der Rest

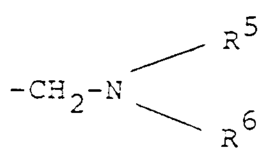

sich in 2-Stellung zur sekundären Aminogruppe befindet, sind beispielsweise aus 4-Benzyl-3-(hydroxymethyl)morpholin (G.R. Brown, A.J. Foubister und B. Wright, J.Chem.Soc. Perkin Trans. I 1985, 2577) durch Überführung in das 4-Benzyl-3-(chlormethyl)morpholin-hydrochlorid unter der Einwirkung von Thionylchlorid, sowie nachfolgende Umsetzung mit Aminen der allgemeinen Formel II und abschließende hydrogenolytische Abspaltung der Schutzgruppe, zugänglich.

Die Ausgangsverbindungen der allgemeinen Formel IV sind bekannt oder werden in Analogie zu den in DE-B-3 127 849.3 und DE-B-1 179 943 genannten Verfahren hergestellt.

Die Ausgangsverbindungen der allgemeinen Formel V, in der Nu eine Alkoxygruppe bedeutet, erhält man durch Umsetzung von Diaminen der allgemeinen Formel III mit Halogenessigsäureestern, gegebenenfalls unter Verwendung zusätzlicher Hilfsbasen, z.B. Triethylamin, oder Katalysatoren, beispielsweise Triton B. Durch Verseifung der erhaltenen Ester, z.B. mit Barytlauge, erhält man die unter die allgemeine Formel V fallenden Carbonsäuren, die zur Herstellung von Derivaten mit anderen nucleofugen Gruppen dienen können.

Ein weiterer Gegenstand der Erfindung sind Arzneimittel, die ein oder mehrere substituierte 6H-Pyrido-[2,3-b][l,4]benzodiazepin-6-one der allgemeinen Formel I bzw. deren physiologisch verträgliche Salze enthalten.

Die Verbindungen der allgemeinen Formel I lassen sich hierzu in an sich bekannter Weise in die üblichen pharmazeutischen Zubereitungsformen, z.B. in Lösungen, Suppositorien, Tabletten, Dragées, Kapseln oder Teezubereitungen einarbeiten. Die Tagesdosis liegt im allgemeinen zwischen 0,02 und 5 mg/kg, vorzugsweise 0,02 und 2,5 mg/kg, insbesondere 0,05 und l,0 mg/kg Körpergewicht, die gegebenenfalls in Form mehrerer, vorzugsweise l bis 3 Einzelgaben, zur Erzielung der gewünschten Ergebnisse verabreicht wird.

Die basisch substituierten kondensierten Diazepinone der allgemeinen Formel I und ihre Säureadditionssalze besitzen wertvolle Eigenschaften; insbesondere besitzen sie günstige Effekte auf die Herzfrequenz und sind angesichts fehlender magensäuresekretionshemmender, salivationshemmender und mydriatischer Einflüsse als vagale Schrittmacher zur Behandlung von Bradycardien und Bradyarrhythmien in der Human- und auch der Veterinärmedizin geeignet; ein Teil der Verbindungen zeigt auch spasmolytische Eigenschaften auf periphere Organe, insbesondere Colon und Blase.

Eine günstige Relation zwischen tachycarden Wirkungen einerseits und den bei Therapeutika mit anticholinerger Wirkkomponente auftretenden unerwünschten Wirkungen auf die Pupillenweite und Tränen-, Speichel- und Magensäuresekretion andererseits ist für die therapeutische Verwendung der Substanzen von besonderer Wichtigkeit. Die folgenden Versuche zeigen, daß die erfindungsgemäßen Verbindungen diesbezüglich überraschend günstige Relationen aufweisen.

A. Untersuchung auf funktionelle Selektivität der anti muscarinischen Wirkung

Substanzen mit antimuscarinischen Eigenschaften inhibieren die Wirkungen von exogen zugeführten Agonisten oder von Acetylcholin, das aus cholinergen Nervenendigungen freigesetzt wird. Im folgenden wird eine Beschreibung von Methoden wiedergegeben, die zur Erfassung von cardioselektiven Antimuscarinica geeignet sind.

"In vitro" Organpräparationen

Dissoziationskonstanten ($K_B$-Werte) wurden "in vitro" am Ileum und spontan schlagenden Vorhof des Meerschweinchens ermittelt. Das Ileum wurde entnommen und im Organbad in Krebs-Henseleit-Lösung inkubiert. Kontraktionen wurden derart durch steigende Konzentrationen von Methacholin (M) hervorgerufen, daß volle Konzentrations-Wirkungskurven aufgenommen werden konnten. Danach wurde M ausgewaschen, die zu untersuchende Substanz beigefügt und 30 Minuten lang in Kontakt gelassen, und wiederum eine Konzentrations-Wirkungskurve mit M aufgenommen.

Aus dem Dosisverhältnis (DR), das ist das Ausmaß der Verschiebung der Konzentrations-Wirkungskurve, wurde die Dissoziationskonstante nach Arunlakshana und Schild (Brit. J. Pharmacol. l4, 48, l959) berechnet.

Am isolierten, spontan schlagenden rechten Vorhof reduzierte M konzentrationsabhängig die Herzfrequenz. Durch Zugabe eines Antimuscarinicums wurde dieser Effekt wieder aufgehoben. Dissoziationskonstanten für die muscarinischen Rezeptoren des Vorhofes wurden auf die gleiche Art und Weise wie oben beschrieben gewonnen. Der Vergleich der in beiden Geweben ermittelten Dissoziationskonstanten erlaubte die Identifizierung von cardioselektiven Substanzen. Die Ergebnisse sind in der Tabelle IV enthalten.

"In vivo" Methoden

Die angewandten Methoden hatten zum Ziel, die Selektivität der antimuscarinischen Wirkung zu bestätigen. Jene Substanzen, die auf der Basis von "in vitro" Untersuchungen ausgewählt worden waren, wurden auf ihre

l. Tachycarde Wirkung am wachen Hund,
2. $M_1$-/$M_2$-Selektivität an der Ratte und
3. Speichselsekretionshemmende Wirkung an der Ratte untersucht.

l. Herzfrequenz steigernde Wirkung am wachen Hund

Die Substanzen wurden entweder intravenös injiziert oder oral verabreicht und die Herzfrequenz mit Hilfe eines Tachygraphen gemessen. Nach einem Kontrollzeitraum wurden steigende Dosen der Verbindung

appliziert, um die Herzfrequenz zu erhöhen. Es wurde jeweils dann die nächste Dosis appliziert, wenn der Effekt der vorangegangenen nicht mehr sichtbar war. Die Dosis einer Substanz, die eine Erhöhung um 50 Schläge/Minute ($ED_{50}$) bewirkte, wurde graphisch ermittelt. Jede Substanz wurde an 3 bis 5 Hunden untersucht. Die Ergebnisse sind aus der Tabelle II zu ersehen.

2. $M_1$-/$M_2$-Selektivität an der Ratte

Die angewandte Methode wurde von Hammer und Giachetti (Life Sciences 3l, 299l-2998 (l982)) beschrieben. 5 Minuten nach intravenöser Injektion steigender Dosen der Substanz wurden entweder der rechte Vagus elektrisch stimuliert (Frequenz: 25 Hz; Pulsbreite: 2ms; Reizdauer: 30s; Voltzahl: supramaximal) oder 0,3 mg/kg McN-A-343 in männliche THOM-Ratten intravenös injiziert. Die durch Vagusstimulation hervorgerufene Bradykardie und der durch McN-A-343 verursachte Blutdruckanstieg wurden bestimmt. Die Dosis der Substanzen, die entweder die vagale Bradykardie ($M_2$) oder den Blutdruckanstieg ($M_1$) um 50% verminderte, wurde graphisch ermittelt. Ergebnisse siehe Tabelle III.

3. Speichselsekretionshemmende Wirkung an der Ratte

Nach Lavy und Mulder (Arch. int. Pharmacodyn. l78, 437-445, (l969)) erhielten mit l,2 g/kg Urethan narkotisierte männliche THOM-Ratten steigende Dosen der Substanz i.v.. Die Speichelsekretion wurde durch s.c. Gabe von 2 mg/kg Pilocarpin ausgelöst. Der Speichel wurde mit Fließpapier aufgesaugt, die von ihm eingenommene Fläche alle 5 Minuten planimetrisch bestimmt. Die Dosis der Substanz, die das Speichelvolumen um 50% verminderte, wurde graphisch ermittelt. Ergebnisse siehe Tabelle III.

B Bindungsstudien an muscarinischen Rezeptoren:

l.) in vitro: Bestimmung des $IC_{50}$-Wertes

Als Organspender dienten männliche Sprague-Dawley-Ratten mit l80-220 g Körpergewicht. Nach Entnahme von Herz, Submandibularis und Großhirnrinde wurden alle weiteren Schritte in eiskaltem Hepes-HCl-Puffer (pH 7,4; l00 m molar NaCl, l0 m molar $MgCl_2$) vorgenommen. Das Gesamtherz wurde mit einer Schere zerkleinert. Alle Organe wurden abschließend in einem Potter homogenisiert.

Für den Bindungstest wurden die Organhomogenate in folgender Weise verdünnt:

Gesamtherz l: 400
Großhirnrinde l:3000
Submandibularis l: 400

Die Inkubation der Organhomogenate erfolgte bei einer bestimmten Konzentration des Radioliganden und einer Konzentrationsreihe der nichtradioaktiven Testsubstanzen im Eppendorf-Zentrifugenröhrchen bei 30° C. Die Inkubationsdauer betrug 45 Minuten. Als Radioligand wurde 0,3 n molar $^3$H-N-Methylscopolamin ($^3$H-NMS) verwendet. Die Inkubation wurde durch Zugabe von eiskaltem Puffer mit nachfolgender Vakuumfiltration beendet. Die Filter wurden mit kaltem Puffer gespült und ihre Radioaktivität bestimmt. Sie repräsentiert die Summe von spezifischer und unspezifischer Bindung von $^3$H-NMS. Der Anteil der unspezifischen Bindung wurde definiert als jene Radioaktivität, die in Anwesenheit von l $\mu$ molar Chinuclidinylbenzilat gebunden wurde. Es wurden immer Vierfachbestimmungen vorgenommen. Die $IC_{50}$-Werte der nichtmarkierten Testsubstanzen wurden graphisch bestimmt. Sie repräsentieren jene Konzentration der Testsubstanz, bei welcher die spezifische Bindung von $^3$H-NMS an die muscarinischen Rezeptoren in den verschiedenen Organen um 50 % gehemmt wurde. Die Ergebnisse sind aus der Tabelle l zu ersehen.

2.) in vivo: Bestimmung der $ID_{50}$-Werte

Für diese Experimente wurden weibliche Ratten mit einem Körpergewicht von ca. 200 g verwendet. Vor Versuchsbeginn wurden die Tiere mit einer Dosis von l,25 g/kg Urethan narkotisiert. Die Tiere erhielten jeweils die vorgesehene Dosis der Prüfsubstanz durch i.v. Injektion. Nach Ablauf von l5 Minuten wurde auf gleiche Weise ll3 ng/kg 3H-N-Methylscopolamin (3H-NMS) gegeben. Nach weiteren l5 Minuten wurden die Tiere getötet, das Herz, die Bronchien und die Tränendrüsen entnommen. Diese Organe wurden in Soluene R aufgelöst und die Radioaktivität bestimmt. Diese Messwerte wurden als Totalbindung angenommen. Der Anteil an unspezifischer Bindung wurde als jene Radioaktivität, die durch Gabe von 2 mg/kg Atropin nicht verdrängbar war, definiert. Aus diesen Versuchen wurden für die einzelnen Organe $ID_{50}$-Werte ermittelt. Die $ID_{50}$-Werte sind Dosen der Prüfsubstanzen, die 50% der spezifischen Bindung von 3H-NMS an die

muskarinischen Rezeptoren der jeweiligen Organe hemmten. Die Ergebnisse sind in der Tabelle V enthalten:

Nach den vorstehenden Angaben wurden beispielsweise die folgenden Verbindungen untersucht:

A = 9-Chlor-ll-[[2-[(diethylamino)methyl]-l-piperidinyl]acetyl]-5,ll-dihydro-6H-pyrido[2,3-b][l,4]-benzodiazepin-6-on

und als Vergleichssubstanzen

B = ll-[[2-[(Diethylamino)methyl]-l-piperidinyl]acetyl]-5,ll-dihydro-6H-pyrido[2,3-b][l,4]benzodiazepin-6-on (siehe US-Patent Nr. 4 550 l07)

C = 5,ll-Dihydro-ll-[(4-methyl-l-piperazinyl)acetyl]-6H-pyrido[2,3-b][l,4]benzodiazepin-6-on (Pirenzepin, siehe US-Patent Nr. 3 660 380)

und

D = Atropin

Tabelle I:

Rezeptor-Bindungs-Tests, in vitro:
Ergebnisse:

| Substanz | Rezeptor-Bindungs-Tests $IC_{50}[nMl^{-1}]$ Cortex | Herz | Submandibularis |
|---|---|---|---|
| A | 500 | 50 | 1500 |
| B | 1200<br>3000 | 140<br>150 | 5000 |
| C | 100 | 1500 | 200 |
| D | 2 | 4 | 4 |

Tabelle II:

Herzfrequenz steigernde Wirkung am wachen Hund:
Ergebnisse:

| Substanz | Tachycardie (Hund) $ED_{50}[\mu g/kg]$ intravenös | oral | Verhältnis $ED_{50}$ p.o./ $ED_{50}$ i.v. |
|---|---|---|---|
| A | 170 | 380 | 2 |
| B | 120 | 1750 | 15 |

Tabelle III:

$M_1/M_2$-Selektivität und speichelsekretionshemmende Wirkung an der Ratte:

Ergebnisse:

| Substanz | $M_2$-Aktivität (Ratte) $ED_{50}$[µg/kg]i.v. | $M_1$-Aktivität (Ratte) $ED_{50}$[µg/kg]i.v. | Speichelsekretionshemmung (Ratte) $ED_{50}$[µg/kg]i.v. |
|---|---|---|---|
| A | 77 | 1476 | 7568 |
| B | 160 | 988 | 4215 |
| C | 883 | 40 | 84 |
| D | 4 | 16 | 9 |

Tabelle IV

| Dissoziationskonstanten ($K_B$-Werte) am Ileum und spontan schlagenden Vorhof des Meerschweinchens: | | |
|---|---|---|
| Ergebnisse: | | |
| Substanz | $K_B$ [mol/l] | |
| | Herz | Ileum |
| A | $1,48 \times 10^{-8}$ | $5,13 \times 10^{-7}$ |
| B | $1,05 \times 10^{-7}$ | $6,17 \times 10^{-7}$ |
| C | $2,4 \times 10^{-7}$ | $1,55 \times 10^{-7}$ |
| D | $1,41 \times 10^{-9}$ | $8,13 \times 10^{-10}$ |

Tabelle V

| Rezeptor-Bindungstests, in vivo: | | | | |
|---|---|---|---|---|
| Ergebnisse: | | | | |
| Substanz | $ID_{50}$ [mg/kg] | | | |
| | Herz | | Bronchien | Tränendrüsen |
| | Atrium | Ventrikel | | |
| A | 0,6 | 0,3 | 8,0 | > 30,0 |
| B | 1,0 | 0,6 | 15,0 | > 30,0 |
| C | 5,0 | 1,0 | 10,0 | 10,0 |
| D | 0,08 | 0,03 | 0,1 | 0,2 |

Die Angaben in der vorstehenden Tabelle I beweisen, daß die neuen Verbindungen der allgemeinen Formel I zwischen muscarinischen Rezeptoren verschiedener Gewebe unterscheiden. Dies folgt aus den beträchtlich niedrigeren $IC_{50}$-Werten bei Untersuchung an Präparaten aus dem Herzen gegenüber solchen aus der Großhirnrinde und Submandibularis.

Aus den pharmakologischen Daten der vorstehenden Tabelle III ergibt sich - in völliger Übereinstimmung mit den Rezeptor-Bindungs-Studien -, daß die Herzfrequenz durch die genannten Verbindungen bereits bei Dosierungen gesteigert wird, bei denen noch keine Einschränkung der Speichselsekretion beobachtet wird. Außerdem deuten die pharmakologischen Daten der vorstehenden Tabelle IV auf ein überraschend großes Unterscheidungsvermögen zwischen Herz und glatter Mukulatur. Die genannten Verbindungen werden hervorragend resorbiert, da sie - wie aus Tabelle II hervorgeht - nach oraler Gabe fast genau so wirksam sind wie nach intravenöser Injektion.

Die Tabelle V zeigt die bevorzugte Bindung an Rezeptoren des Herzens (Atrium/Ventrikel).

Ferner sind die erfindungsgemäß hergestellten Verbindungen gut verträglich, so konnten selbst bei den höchsten applizierten Dosen bei den pharmakologischen Untersuchungen keine toxischen Nebenwirkungen beobachtet werden.

Die nachfolgenden Beispiele sollen die Erfindung näher erläutern:

"Fp." bedeutet "Schmelzpunkt", "Z." bedeutet "Zersetzung". Für alle Verbindungen liegen befriedigende Elementaranalysen, IR-, UV-, $^1$H-NMR-, häufig auch Massenspektren vor.

Herstellung der Ausgangsmaterialien

Beispiel A

2-Chlor-ll-(chloracetyl)-5,ll-dihydro-6H-pyrido[2,3-b][l,4]benzodiazepin-6-on

9,08 g (0,04 Mol) 5,ll-Dihydro-6H-pyrido[2,3-b][l,4]benzodiazepin-6-on-l-oxid (CRC Compagnia di Ricerca Chimica S.p.A., S. Giovanni al Natisone DE-A-3 208 656) wurden in l50 ml trockenem Acetonitril suspendiert und nach Zugabe von 42,6 g (0,37 Mol) Chloracetylchlorid 2 Stunden lang auf eine Innentemperatur von 76°C erwärmt. Anschließend kühlte man auf 0°C ab, nutschte den entstandenen Niederschlag nach 2-stündigem Stehenlassen bei dieser Temperatur ab und wusch ihn mit l5 ml kaltem Acetonitril nach. Die Mutterlauge wurde im Vakuum eingedampft, der ölige Rückstand mit einer Mischung aus 20 ml Acetonitril und 50 ml Wasser gründlich verrieben, der kristalline Niederschlag abgesaugt und mit l00 ml Wasser gewaschen. Beide Fraktionen wurden vereinigt und bei 40°C im Vakuum getrocknet. Ausbeute: ll,64 g (90% der Theorie). Zur Reinigung wurde aus Ethanol umkristallisiert. Farblose Kristalle vom Fp. 235-238°C (Z.).

Beispiel B

9-Chlor-ll-(chloracetyl-5,ll-dihydro-6H-pyrido[2,3-b][l,4]benzodiazepin-6-on

Zu der siedenden Mischung aus 8l,7 g (0,333 Mol) 9-Chlor-5,ll-dihydro-6H-pyrido[2,3-b][l,4]-

benzodiazepin-6-on, l660 ml wasserfreiem Dioxan und 75 ml (0,536 Mol) Triethylamin tropfte man innerhalb von 30 Minuten 40 ml (0,529 Mol) Chloracetylchlorid und rührte anschließend noch 4 Stunden bei Rückflußtemperatur. Die noch heiße Mischung wurde filtriert, der Nutschenrückstand mit eiskaltem Wasser gründlich gewaschen und mit dem Produkt vereinigt, das man durch Einengen des Dioxan-haltigen Filtrats und Verreiben des Rückstandes mit Wasser erhalten hatte. Nach dem Trocknen im Vakuum kristallisierte man aus Dimethylformamid. Farblose Kristalle vom Fp. 228-230 ° C. Ausbeute: 96 g (89% der Theorie).

Entsprechend wurden erhalten:

ll-(Chloracetyl)-5,ll-dihydro-2-methyl-6H-pyrido[2,3-b][l,4]benzodiazepin-6-on vom Fp. 202-204 ° C (Z.) (aus Xylol);

8-Chlor-ll-(chloracetyl)-5,ll-dihydro-6H-pyrido[2,3-b][l,4]benzodiazepin-6-on vom Fp. 2ll-2l2 ° C (Z.) (aus Ethanol);

ll-(Chloracetyl)-5,ll-dihydro-8-methyl-6H-pyrido[2,3-b][l,4]benzodiazepin-6-on vom Fp. 233-235 ° C (aus Ethoxyethanol);

ll-Chloracetyl)-5,ll-dihydro-2,4,l0-trimethyl-6H-pyrido[2,3-b][l,4]benzodiazepin-6-on vom Fp. l5l-l53 ° C (Acetonitril);

ll-Chloracetyl)-5,ll-dihydro-2,4-dimethyl-6H-pyrido[2,3-b][l,4]benzodiazepin-6-on vom Fp. l94-l95 ° C (Z.) (Acetonitril);

ll-(Chloracetyl)-5,ll-dihydro-2,l0-dimethyl-6H-pyrido[2,3-b][l,4]benzodiazepin-6-on vom Fp. 230-232 ° C (Z.) (aus Isopropanol);

ll-(Chloracetyl)-5,ll-dihydro-l0-methyl-6H-pyrido[2,3-b][l,4]benzodiazepin-6-on vom Fp. 2l9-220 ° C (aus Acetonitril);

ll-(Chloracetyl)-5,ll-dihydro-2,8-dimethyl-6H-pyrido[2,3-b][l,4]benzodiazepin-6-on;

ll-(Chloracetyl-2,9-dichlor-5,ll-dihydro-6H-pyrido[2,3-b][l,4]benzodiazepin-6-on;

ll-(Chloracetyl)-5,ll-dihydro-8,9-dimethyl-6H-pyrido[2,3-b][l,4]benzodiazepin-6-on;

ll-(Chloracetyl)-5,ll-dihydro-8-ethyl-6H-pyrido[2,3-b][l,4]benzodiazepin-6-on vom Fp. 200 bis 20l ° C;

ll-(Chloracetyl)-5,ll-dihydro-9-methyl-6H-pyrido[2,3-b][l,4]benzodiazepin-6-on vom Fp. 205 ° C (Z.);

8-Brom-ll-(chloracetyl)-5,ll-dihydro-6H-pyrido[2,3-b][l,4]benzodiazepin-6-on vom Fp. 222 ° C (Z.);

9-Brom-ll-(chloracetyl)-5,ll-dihydro-6H-pyrido[2,3-b][l,4]benzodiazepin-6-on;

ll-(Chloracetyl)-5,ll-dihydro-7-fluor-6H-pyrido[2,3-b][l,4]benzodiazepin-6-on;

ll-(Chloracetyl)-5,ll-dihydro-8-fluor-6H-pyrido[2,3-b][l,4]benzodiazepin-6-on;

ll-(Chloracetyl)-5,ll-dihydro-9-fluor-6H-pyrido[2,3-b][l,4]benzodiazepin-6-on;

ll-(Chloracetyl)-5,ll-dihydro-2,4,8-trimethyl-6H-pyrido[2,3-b][l,4]benzodiazepin-6-on vom Fp. > 230 ° C (Isopropanol).

## Beispiel C

### 3-[(Diethylamino)methyl]pyrrolidin

Zu der Suspension von 26,0 g (0,ll9 Mol) l-Benzyl-2,3-dihydro-5(4H)-oxo-lH-pyrrol-3-carbonsäure in 200 ml wasserfreiem Tetrahydrofuran gab man 9,43 ml (0,l3l Mol) Thionylchlorid und hielt die Mischung unter Rühren l Stunde bei 45 ° C. Die erhaltene klare Lösung wurde im Vakuum eingedampft, der Rückstand in 200 ml frischem Tetrahydrofuran aufgenommen und tropfenwese mit der Lösung von 36,5 ml (0,355 Mol) Diethylamin in 200 ml trockenem Tetrahydrofuran versetzt. Die Mischung wurde 3 Stunden unter Rückfluß gekocht, dann nach dem Erkalten vom ausgefallenen Diethylamin-hydrochlorid befreit. Das Filtrat wurde eingedampft, das zurückbleibende Öl in 200 ml einer Mischung aus 2 Teilen Diethylether und l Teil Essigsäureethylester aufgenommen, mit 3 g Aktivkohle behandelt und erneut eingedampft. Das zurückbleibende viskose, gelbliche Produkt (30,0 g, 92% der Theorie) wurde ohne weitere Reinigung in 500 ml wasserfreiem Tetrahydrofuran aufgenommen und zu der Suspension von 8,36 g (0,22 Mol) Lithiumaluminiumhydrid in 500 ml Tetrahydrofuran getropft. Man kochte noch 2 Stunden unter Rückfluß, ließ erkalten und versetzte nacheinander mit 9 ml Wasser, 9 ml l5 proz. wässeriger Natronlauge und 27 ml Wasser. Man filtrierte und dampfte das Filtrat im Vakuum ein. Der erhaltene ölige Rückstand (25 g, 93% der Theorie) wurde in l00 ml Ether gelöst und ins Hydrochlorid übergeführt. Die Lösung dieses Hydrochlorids in 350 ml Methanol wurde in Gegenwart von l2 g l0 proz. Palladium/Tierkohle bei Zimmertemperatur und Normaldruck bis zur Beendigung der Wasserstoff-Aufnahme hydriert. Man arbeitete wie üblich auf und erhielt l2,5 g (79% der Theorie; Gesamtausbeute: 67% der Theorie) eines farblosen Öls vom $Sdp._{12\ mm\ Hg}$ 88-93 ° C.

## Beispiel D

3-[(Diethylamino)methyl]morpholin

Zu der Lösung von l6,6 g (0,08 Mol) 4-Benzyl-3-(hydroxymethyl)-morpholin in l00 ml wasserfreiem Dichlormethan tropfte man 20,2 g (0,l7 Mol) Thionylchlorid, wobei sich die Mischung von selbst erwärmte. Man kochte noch 2 Stunden unter Rückfluß, verrührte die erhaltene tiefdunkelbraune Mischung nach dem Erkalten mit l00 ml Toluol und engte sie im Vakuum ein. Der Rückstand wurde mit l0 ml einer Toluol-Acetonitril-Mischung (l:l v/v) verrieben, wodurch man l8,0 g (86% der Theorie) an farblosen, sehr hygroskopischen Kristallen (4-Benzyl-3-(chlormethyl)morpholin-hydrochlorid) erhielt, die in 30 ml Ethanol gelöst und nach Zusatz von 73,l4 g (l,0 Mol) Diethylamin und l,5 g Natriumiodid 6 Stunden im Autoklaven auf l00 °C erhitzt wurden. Man ließ erkalten, dampfte die Mischung im Vakuum ein und digerierte den verbleibenden Rückstand mit l00 ml heißem t-Butyl-methylether. Man filtrierte, behandelte das Filtrat mit l g Tierkohle, kochte auf und filtrierte erneut. Der nach dem Eindampfen verbleibende ölige Rückstand wurde an 600 g Kieselgel (Macherey-Nagel, 35-70 mesh) unter Verwendung von Dichlormethan/Essigsäureethylester/Cyclohexan/Methanol/konz. Ammoniak 52,8/4l,7/2,6/2,6/0,3 (v/v) säulenchromatographisch gereinigt. Die Fraktion mit $R_F$ 0,6 (Macherey-Nagel, Polygram ® Sil G/$UV_{254}$, pre-coated plastic sheets for TLC, Fließmittel wie vorstehend) wurde isoliert und spektroskopisch und durch Elementaranalyse als das gesuchte 4-Benzyl-3-[(diethylamino)methyl]morpholin identifiziert. Ausbeute: l5,3 g (85% der Theorie). Die Lösung der farblosen Verbindung in 230 ml Ethanol wurde nach Zugabe von 3 Palladiumhydroxid 30 Minuten bei einem Wasserstoffdruck von 5 bar hydriert. Nach Entfernung des Katalysators und üblicher Aufarbeitung erhielt man 7,0 g (70% der Theorie) eines farblosen Öls vom $Sdp._{12}$ 98-ll0 °C (Kugelrohr). Gesamtausbeute über sämtliche Stufen: 5l% der Theorie.

Beispiel E

2-[(Dipropylamino)methyl]piperidin

Die Mischung aus l70,l g (l,0 Mol) 2-(Chlormethyl)piperidin-hydrochlorid (M. Rink und H.G. Liem, Arch. Pharm. 292, l65-l69 (l959)), 506 g (5,0 Mol) Dipropylamin und l,7 l Dichlormethan wurde 3 Stunden unter Rückfluß gekocht, dann im Vakuum eingedampft und mit Kalilauge unter äußerer Kühlung mit Eis alkalisch gestellt. Man extrahierte erschöpfend mit t-Butyl-methylether, wusch die vereinigten Extrakte zweimal mit je l00 ml Wasser, trocknete über Natriumsulfat und entfernte das Lösungsmittel durch Destillation im Vakuum. Der Rückstand wurde im Wasserstrahlvakuum fraktioniert destilliert. Farbloses Öl vom $Sdp._{12}$ mm Hg l08-ll4 °C. Ausbeute: l08,6 g (55% der Theorie).

Beispiel F

9-Chlor-5,ll-dihvdro-6H-pyrido[2,3-b][l,4]benzodiazepin-6-on

Zu der Lösung von l44,0 g (l,l2 Mol) 2-Chlor-3-pyridinamin in 432 ml trockenem Dimethylsulfoxid gab man l39,0 g (l,24 Mol) Kalium-tert.butanolat und rührte l0 Minuten bei einer Reaktionstemperatur von 40 °C. In die entstandene dunkle Lösung wurde innerhalb von l0 Minuten eine Lösung von 207,0 g (l,l2 Mol) 2-Amino-4-chlorbenzoesäuremethylester in 250 ml Dimethylsulfoxid eingetropft, die Mischung anschließend noch 30 Minuten auf 50 °C erwärmt. Man ließ erkalten, rührte in l Liter Eiswasser ein und stellte durch Zugabe von 20proz. wässriger Salzsäure auf pH 4. Der entstandene Kristallbrei wurde abgesaugt, anschließend in l Liter lproz. wässrigem Ammoniak suspendiert und erneut abgenutscht. Nach dem Trocknen im Umlufttrockenschrank: Farblose Kristalle vom Fp. l89-l92 °C, die ohne weitere Reinigung direkt weiter umgesetzt wurden. $R_F$ 0,8 (Macherey-Nagel, Polygram® SIL G/$UV_{254}$, pre-coated plastic sheets for TLC, Fließmittel: Essigsäureethylester/Dichlormethan l:l v/v). Ausbeute 253 g (80% der Theorie).

278,3 g (0,986 Mol) des erhaltenen 2-Amino-4-chlor-N-(2-chlor-3-pyridinyl)benzamids wurden in 436 ml wasserfreiem l,2,4-Trichlorbenzol suspendiert und unter Rühren 8 Stunden auf 220 °C, dann 8 Stunden auf 250 °C erhitzt. Der nach dem Erkalten angefallene Kristallbrei wurde abgenutscht und zweimal mit je 50 ml Trichlorbenzol, dann mit l00 ml Dichlormethan und schließlich mit l00 ml einer Mischung aus 50 ml Ethanol und 50 ml konz. Ammoniak gründlich gewaschen. Man kristallisierte aus 250 ml siedendem Dimethylformamid um, wusch die erhaltenen Kristalle noch je zweimal mit je 50 ml Dimethylformamid und Methanol und trocknete im Umlufttrockenschrank. Ausbeute: 8,4 g, fahlgelbe Kristalle, Fp. >360 °C, $R_F$ 0,70 (Macherey-Nagel, Polygram® SIL G/$UV_{254}$, pre-coated plastic sheets for TLC, Fließmittel: Essigsäureethylester/Dichlormethan/Petrolether 42:42:l6 v/v). Aus den Mutterlaugen konnten durch Eindampfen und gleichartige Aufarbeitung nochmals l7,5 g an Material gleicher Qualität gewonnen werden.

Gesamtausbeute: l0l,5 g (4l,3% der Theorie).

Entsprechend wurden erhalten:
5,ll-Dihydro-2-methyl-6H-pyrido[2,3-b][l,4]benzodiazepin-6-onvom Fp. 257-259 ° C (DMF);
8-Chlor-5,ll-dihydro-6H-pyrido[2,3-b][l,4]benzodiazepin-6-on vom Fp. 307-309 ° C (DMF);
5,ll-Dihydro-8-methyl-6H-pyrido[2,3-b][l,4]benzodiazepin-6-onvom Fp. 257-258 ° C (Diethylenglykoldiethylether);
5,ll-Dihydro-2,4,l0-trimethyl-6H-pyrido[2,3-b][l,4]benzodiazepin-6-on vom Fp. 3l0-3l3 ° C (Ethylglykol);
5,ll-Dihydro-2,4-dimethyl-6H-pyrido[2,3-b][l,4]benzodiazepin-6-on vom Fp. 28l-283 ° C (aus N,N-Dimethylacetamid);
5,ll-Dihydro-2,l0-dimethyl-6H-pyrido[2,3-b][l,4]benzodiazepin-6-on vom Fp. 25l-253 ° C (Xylol);
5,ll-Dihydro-l0-methyl-6H-pyrido[2,3-b][l,4]benzodiazepin-6-on vom Fp. 226-228 ° C (Xylol);
5,ll-Dihydro-2,8-dimethyl-6H-pyrido[2,3-b][l,4]benzodiazepin-6-on vom Fp. 244-246 ° C (Xylol);
2,9-Dichlor-5,ll-dihydro-6H-pyrido[2,3-b][l,4]benzodiazepin-6-on vom Fp. >3l0 ° C;
5,ll-Dihydro-8,9-dimethyl-6H-pyrido[2,3-b][l,4]benzodiazepin-6-on;
5,ll-Dihydro-8-ethyl-6H-pyrido[2,3-b][l,4]benzodiazepin-6-on vom Fp. 232 bis 234 ° C (aus 70 Gew.-% wässriger Essigsäure);
5,ll-Dihydro-9-methyl-6H-pyrido[2,3-b][l,4]benzodiazepin-6-onvom Fp. 286 bis 288 ° C;
8-Brom-5,ll-dihydro-6H-pyrido[2,3-b][l,4]benzodiazepin-6-on vom Fp. 338 bis 340 ° C (Acetonitril);
9-Brom-5,ll-dihydro-6H-pyrido[2,3-b][l,4]benzodiazepin-6-on;
5,ll-Dihydro-7-fluor-6H-pyrido[2,3-b][l,4]benzodiazepin-6-on;
5,ll-Dihydro-8-fluor-6H-pyrido[2,3-b][l,4]benzodiazepin-6-on;
5,ll-Dihydro-9-fluor-6H-pyrido[2,3-b][l,4]benzodiazepin-6-on vom Fp. >330 ° C (Z.);
l0-Chlor-5,ll-dihydro-6H-pyrido[2,3-b][l,4]benzodiazepin-6-onvom Fp. 303-305 ° C (N,N-Dimethylacetamid);
2-Chlor-5,ll-dihydro-6H-pyrido[2,3-b][l,4]benzodiazepin-6-on vom Fp. 276-278 ° C (n-Propanol);
5,ll-Dihydro-2,4,8-trimethyl-6H-pyrido[2,3-b][l,4]benzodiazepin-6-on vom Fp. 228-230 ° C (Xylol).

Herstellung der Endprodukte:

Beispiel l

2-Chlor-ll-[[2-[(diethylamino)methyl]-l-piperidinyl]acetyl]-5,ll-dihydro-6H-pyrido[2,3-b][l,4]benzodiazepin-6-on

Das Gemisch aus 7,l g (0,22 Mol) 2-Chlor-ll-(chloracetyl)-5,ll-dihydro-6H-pyrido[2,3-b][l,4]benzodiazepin-6-on, 30 ml wasserfreiem Dimethylformamid, 4,4 g (0,0258 Mol) D,L-2-[(Diethylamino)methyl]piperidin und 2,4 g (0,0226 Mol) Natriumcarbonat wurde l Stunde bei 60 ° C und 2 Stunden bei 70 ° C Reaktionstemperatur gerührt. Die erkaltete Reaktionsmischung wurde in 300 ml Eiswasser eingerührt, der ausgefallene farblose Niederschlag abgenutscht und aus 50 ml Ethanol unter Verwendung von 0,5 g Tierkohle umkristallisiert. Man erhielt 6,2 g (62% der Theorie) an farblosen Kristallen vom Fp. l9l-l92,5 ° C.

Beispiel 2

9-Chlor-ll-[[2-[(diethylamino)methyl]-l-piperidinyl]acetyl]-5,ll-dihydro-6H-pyrido[2,3-b][l,4]benzodiazepin-6-on

Die Mischung aus l25 g (0,388 Mol) 9-Chlor-ll-(chloracetyl)-5,ll-dihydro-6H-pyrido[2,3-b][l,4]-benzodiazepin-6-on, 2480 ml trockenem Dimethylformamid, 73,0 g (0,429 Mol) D,L-2-[(Diethylamino)-methyl]piperidin, 60 ml (0,429 Mol) wasserfreiem Triethylamin und 3,0 g Natriumiodid wurde 32 Stunden bei einer Innentemperatur von 50 ° C und anschließend l5 Stunden bei Raumtemperatur gerührt. Man filtrierte von Ungelöstem ab und dampfte das Filtrat an der Ölpumpe und einer Badtemperatur von 45 ° C ein. Das zurückbleibende Produkt wurde in 5proz. Salzsäure gelöst und zweimal mit je 50 ml Dichlormethan ausgeschüttelt. Die wässrige Schicht wurde durch Zugabe von konzentrierter Kaliumcarbonatlösung alkalisch gestellt, die freigesetzte Base im Dichlormethan aufgenommen, mit 2 g Aktivkohle behandelt und über Natriumsulfat getrocknet. Das nach dem Abdampfen des Lösungsmittels verbleibende dunkle Öl wurde an 800 g Kieselgel (35-70 mesh) unter Verwendung von Dichlormethan/Methanol/Essigsäureethylester/Cyclohexan/konz. Ammoniak 59/7,5/25/7,5/l (v/v) zum Eluieren säulenchromatographisch gereinigt. Die die gesuchte Verbindung enthaltenden Fraktionen (l32 g) wurden in verdünnter, wässeriger Maleinsäurelösung gelöst. Die filtrierte Lösung wurde viermal mit je 50 ml Essigsäureethylester gewaschen, dann mit festem Kaliumcarbonat gesättigt und erschöpfend mit Dichlormethan extrahiert. Die vereinigten Dichlormethanphasen wurden über Natriumsulfat getrocknet und eingedampft und

hinterließen l09 g eines teilweise kristallisierenden Öls. Nach zweimaligem Umkristallisieren aus je 450 ml Acetonitril unter Verwendung von jeweils 2 g Aktivkohle erhielt man 63,0 g (36% der Theorie) an farblosen Kristallen vom Fp. l67,5-l69 ° C.

Beispiel 3

ll-[[2-[(Diethylamino)methyl]-l-piperidinyl]acetyl]-5,ll-dihydro-8-methyl-6H-pyrido[2,3-b][l,4]benzodiazepin-6-on

Hergestellt analog Beispiel 2 aus ll-(Chloracetyl)-5,ll-dihydro-8-methyl-6H-pyrido[2,3-b][l,4]-benzodiazepin-6-on und 2-[(Diethylamino)methyl]piperidin, jedoch unter Verwendung von Acetonitril als Lösungsmittel, in einer Ausbeute von 54% der Theorie. Fp. l73-l74 ° C (Diisopropylether).

Beispiel 4

ll-[[2-[(Diethylamino)methyl]-l-piperidinyl]acetyl]-5,ll-dihydro-2-methyl-6H-pyrido[2,3-b][l,4]benzodiazepin-6-on

Hergestellt analog Beispiel 2 aus ll-(Chloracetyl)-5,ll-dihydro-2-methyl-6H-pyrido[2,3-b][l,4]-benzodiazepin-6-on und 2-[(Diethylamino)methyl]piperidin, jedoch unter Verwendung von Acetonitril als Lösungsmittel, in einer Ausbeute von 65% der Theorie. Fp. l84-l86 ° C (nach Umkristallisieren aus Diisopropylether und Acetonitril, jeweils unter Verwendung von Aktivkohle).

Entsprechend wurden erhalten:

ll-[[2-[(Diethylamino)methyl]-l-piperidinyl]acetyl]-5,ll-dihydro-9-methyl-6H-pyrido[2,3-b][l,4]benzodiazepin-6-on vom Fp. l72 bis l74 ° C (Acetonitril);
8-Chlor-ll-[[2-[(diethylamino)methyl]-l-piperidinyl]acetyl]-5,ll-dihydro-6H-pyrido[2,3-b][l,4]benzodiazepin-6-on vom Fp. l86 bis l88 ° C (Acetonitril unter Verwendung von Aktivkohle);
ll-[[2-[(Diethylamino)methyl]-l-piperidinyl]acetyl]-5,ll-dihydro-2,4,l0-trimethyl-6H-pyrido[2,3-b][l,4]-benzodiazepin-6-on;
ll-[[2-[(Diethylamino)methyl]-l-piperidinyl]acetyl]-5,ll-dihydro-2,4,8-trimethyl-6H-pyrido[2,3-b][l,4]-benzodiazepin-6-on vom Fp. 2l3 bis 2l5 ° C (Acetonitril);
ll-[[2-[(Diethylamino)methyl]-l-piperidinyl]acetyl]-5,ll-dihydro-2,8-dimethyl-6H-pyrido[2,3-b][l,4]benzodiazepin-6-on;
2,9-Dichlor-ll-[[2-[(diethylamino)methyl]-l-piperidinyl]acetyl]-5,ll-dihydro-6H-pyrido[2,3-b][l,4]benzodiazepin-6-on;
ll-[[2-[(Diethylamino)methyl]-l-piperidinyl]acetyl]-5,ll-dihydro-8-fluor-6H-pyrido[2,3-b][l,4]benzodiazepin-6-on;
ll-[[2-[(Diethylamino)methyl]-l-piperidinyl]acetyl]-5,ll,dihydro-9-fluor-6H-pyrido[2,3-b][l,4]benzodiazepin-6-on;
ll-[[2-[(Diethylamino)methyl]-l-piperidinyl]acetyl]-5,ll-dihydro-7-fluor-6H-pyrido[2,3-b][l,4]benzodiazepin-6-on;
8-Brom-ll-[[2-[(diethylamino)methyl]-l-piperidinyl]acetyl]-5,ll-dihydro-6H-pyrido[2,3-b][l,4]benzodiazepin-6-on vom Fp. l65 bis l67 ° C (aus Acetonitril unter Verwendung von Aktivkohle);
ll-[[2-[(Diethylamino)methyl]-l-piperidinyl]acetyl]-5,ll-dihydro-8-ethyl-6H-pyrido[2,3-b][l,4]benzodiazepin-6-on vom Fp. l4l bis l43 ° C (Diisopropylether);
( + )-9-Chlor-ll-[[2-[(diethylamino)methyl]-l-piperidinyl]acetyl]-5,ll-dihydro-6H-pyrido[2,3-b][l,4]benzodiazepin-6-on;
(-)-9-Chlor-ll-[[2-[(diethylamino)methyl]-l-piperidinyl]acetyl]-5,ll-dihydro-6H-pyrido[2,3-b][l,4]benzodiazepin-6-on;
9-Chlor-5,ll-dihydro-ll-[[2-[(dimethylamino)methyl]-l-piperidinyl]acetyl]-6H-pyrido[2,3-b][l,4]benzodiazepin-6-on;
9-Chlor-5,ll-dihydro-ll-[(2-[(l-pyrrolidinyl)methyl]-l-piperidinyl]acetyl]-6H-pyrido[2,3-b][l,4]benzodiazepin-6-on;
9-Chlor-5,ll-dihydro-ll-[(2-[(4-morpholinyl)methyl]-l-piperidinyl]acetyl]-6H-pyrido[2,3-b][l,4]benzodiazepin-6-on;
9-Chlor-ll-[[3-[(diethylamino)methyl]-l-piperidinyl]acetyl]-5,ll-dihydro-6H-pyrido[2,3-b][l,4]benzodiazepin-6-on;
(S)-9-Chlor-ll-[[2-[(diethylamino)methyl]-l-pyrrolidinyl]acetyl]-5,ll-dihydro-6H-pyrido[2,3-b][l,4]benzodiazepin-6-on;
9-Chlor-5,ll-dihydro-ll-[[3-(dimethylamino)-l-piperidinyl]acetyl]-6H-pyrido[2,3-b][l,4]benzodiazepin-6-on;
D,L-9-Chlor-ll-[[3-[(diethylamino)methyl]-4-morpholinyl]acetyl]-5,ll-dihydro-6H-pyrido[2,3-b][l,4]-benzodiazepin-6-on.

Beispiel 5

9-Chlor-ll-[[2-[(diethylamino)methyl]-l-piperidinyl]acetyl]-5,ll-dihydro-6H-pyrido[2,3-b][l,4]benzodiazepin-6-on

5,6 g (0,0l74 Mol) 9-Chlor-ll-(chloracetyl)-5,ll-dihydro-6H-pyrido[2,3-b][l,4]benzodiazepin-6-on wurden in l00 ml wasserfreiem Dioxan suspendiert und nach Zugabe von 6,0 g (0,035 Mol) 2-[(Diethylamino)methyl]-piperidin l2 Stunden unter Rückfluß gekocht.

Die Mischung wurde eingedampft, der Rückstand wie im Beispiel 2 weiterbehandelt. Man erhielt 2,l g (26% der Theorie) an farblosen Kristallen vom Fp. l67,5-l69 ° C (Acetonitril/Aktivkohle).

Beispiel 6

9-Chlor-ll-[[2-[(diethylamino)methyl]-l-piperidinyl]acetyl]-5,ll-dihydro-6H-pyrido[2,3-b][l,4]benzodiazepin-6-on

Man erwärmte ein Gemisch von l4,43 g (0,0632 Mol) 2-[(Diethylamino)methyl]-l-piperidinessigsäure und 2,0 g einer 75proz. Natriumhydrid-Dispersion in Paraffinöl in l60 ml Dimethylformamid bei 50 bis 80 ° C so lange, bis die Wasserstoffentwicklung beendet war. Zu dem entstandenen Natriumsalz der genannten Säure fügte man l5,35 g (0,0625 Mol) 9-Chlor-5,ll-dihydro-6H-pyrido[2,3-b][l,4]benzodiazepin-6-on und tropfte bei -l0 ° C 9,9 g (0,0646 Mol) Phosphoroxidchlorid innerhalb von l0 Minuten zu. Man rührte 4 Stunden bei -l0 ° C, 4 Stunden bei 0 ° C und 20 Stunden bei Zimmertemperatur. Man rührte die Mischung in 300 g Eis ein, stellte mit Natronlauge auf pH 9 und schüttelte erschöpfend mit Dichlormethan aus. Die vereinigten organischen Phasen wurden einmal mit wenig Eiswasser gewaschen, über Natriumsulfat getrocknet und eingedampft. Der Rückstand wurde aus Acetonitril unter Verwendung von Aktivkohle umkristallisiert. Farblose Kristalle vom Fp. l67,5-l69 ° C, nach Dünnschichtchromatogramm, Mischschmelzpunkt, IR-, UV- und $^1$H-NMR-Spektrum völlig identisch mit einer nach Beispiel 2 erhaltenen Probe. Ausbeute: 5,l g (l8 % der Theorie).

Im folgenden wird die Herstellung pharmazeutischer Anwendungsformen anhand einiger Beispiele beschrieben:

Beispiel I

Tabletten mit 5 mg 9-Chlor-11-[[2-[(diethylamino)methyl]-1-piperidinyl]acetyl]-5,11-dihydro-6H-pyrido[2,3-b]-[1,4]-benzodiazepin-6-on

| Zusammensetzung: | |
|---|---|
| 1 Tablette enthält: | |
| Wirkstoff | 5,0 mg |
| Milchzucker | 148,0 mg |
| Kartoffelstärke | 65,0 mg |
| Magnesiumstearat | 2,0 mg |
| | 220,0 mg |

Herstellungsverfahren:

Aus Kartoffelstärke wird durch Erwärmen ein 10%iger Schleim hergestellt. Die Wirksubstanz, Milchzucker und die restliche Kartoffelstärke werden gemischt und mit obigem Schleim durch ein Sieb der Maschenweite 1,5 mm granuliert. Das Granulat wird bei 45 ° C getrocknet, nochmals durch obiges Sieb gerieben, mit Magnesiumstearat vermischt und zu Tabletten verpreßt.

Tablettengewicht: 220 mg
Stempel: 9 mm

Beispiel II

Dragées mit 5 mg 9-Chlor-11-[[2-[(diethylamino)methyl]-1-piperidinyl]acetyl]-5,11-dihydro-6H-pyrido[2,3-b]-

[1,4]-benzodiazepin-6-on

Die nach Beispiel I hergestellten Tabletten werden nach bekanntem Verfahren mit einer Hülle überzogen, die im wesentlichen aus Zucker und Talkum besteht. Die fertigen Dragées werden mit Hilfe von Bienenwachs poliert.
Dragéegewicht: 300 mg

Beispiel III

Ampullen mit 10 mg 9-Chlor-11-[[2-[(diethylamino)methyl]-1-piperidinyl]acetyl]-5,11-dihydro-6H-pyrido[2,3-b][1,4]-benzodiazepin-6-on

| Zusammensetzung: | | |
|---|---|---|
| 1 Ampulle enthält: | | |
| Wirkstoff | | 10,0 mg |
| Natriumchlorid | | 8,0 mg |
| Dest. Wasser | ad | 1 ml |

Herstellungsverfahren:

Die Wirksubstanz und Natriumchlorid werden in dest. Wasser gelöst und anschließend auf das gegebene Volumen aufgefüllt. Die Losung wird sterilfiltriert und in 1 ml-Ampullen abgefüllt.
Sterilisation: 20 Minuten bei 120° C.

Beispiel IV

Suppositorien mit 20 mg 9-Chlor-11-[[2-[(diethylamino)-methyl]-1-piperidinyl]acetyl]-5,11-dihydro-6H-pyrido-[2,3-b][1,4]benzodiazepin-6-on

| Zusammensetzung: | |
|---|---|
| 1 Zäpfchen enthält: | |
| Wirkstoff | 20,0 mg |
| Zäpfchenmasse (z.B. Witepsol W 45$^{(R)}$) | 1 680,0 mg |
| | 1 700,0 mg |

Herstellungsverfahren:

Die feinpulverisierte Wirksubstanz wird in der geschmolzenen und auf 40° C abgekühlten Zäpfchenmasse suspendiert. Man gießt die Masse bei 37° C in leicht vorgekühlte Zäpfchenformen aus.
Zäpfchengewicht l,7 g

Beispiel V

Tropfen mit 4,9-Dihydro-9-chlor-ll-[[2-[(diethylamino)methyl]-l-piperidinyl]acetyl]-5,ll-dihydro-6H-pyrido[2,3-b][l,4]benzodiazepin-6-on

Zusammensetzung:
l00 ml Tropflösung enthalten:
p-Hydroxybenzoesäuremethylester 0,035 g
p-Hydroxybenzoesäurepropylester 0,0l5 g

Anisöl 0,05 g
Menthol 0,06 g
Ethanol rein I0,0 g
Wirkstoff 0,5 g
Natriumcyclamat I,0 g
Glycerin I5,0 g
Dest. Wasser ad I00,0 ml

Herstellungsverfahren:

Die Wirksubstanz und Natriumcyclamat werden in ca. 70 ml Wasser gelöst und Glycerin zugefügt. Man löst p-Hydroxybenzoesäureester, Anisöl sowie Menthol in Ethanol und fügt diese Lösung unter Rühren der wäßrigen Lösung zu. Abschließend wird mit Wasser auf I00 ml aufgefüllt und schwebeteilchenfrei filtriert.

## Patentansprüche

## Patentansprüche für folgende Vertragsstaaten : BE, CH, DE, FR, GB, IT, LU, NL, SE

**1.** Substituierte Pyrido[2,3-b][I,4]benzodiazepin-6-one der allgemeinen Formel I

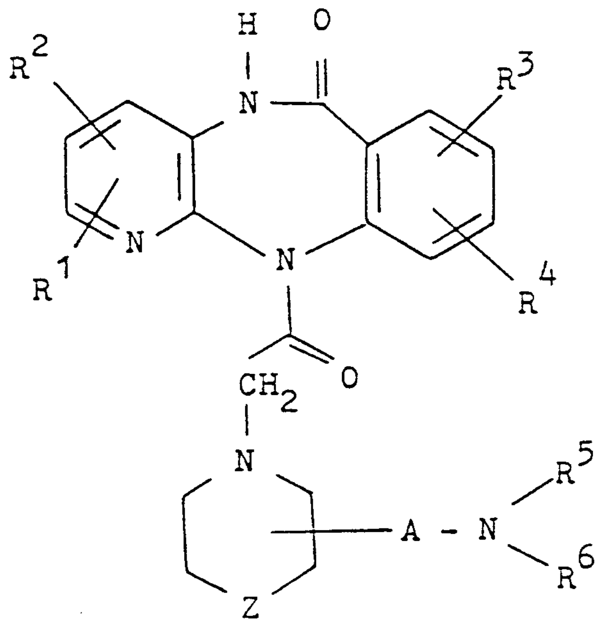

(I),

in der
$R^1$ eine Alkylgruppe mit I bis 4 C-Atomen, ein Chloratom oder ein Wasserstoffatom;
$R^2$ ein Wasserstoffatom oder eine Methylgruppe;
$R^3$ und $R^4$ jeweils ein Wasserstoffatom, ein Fluor-, Chloroder Bromatom oder eine Alkylgruppe mit I bis 4 C-Atomen bedeuten, jedoch mit der Maßgabe, daß wenigstens einer der Reste $R^1$, $R^2$, $R^3$ und $R^4$ von Wasserstoff verschieden ist,
$R^5$ und $R^6$ gleiche oder voneinander verschiedene Alkylreste mit bis zu 6 Kohlenstoffatomen darstellen oder zusammen mit dem dazwischenliegenden Stickstoffatom einen 4- bis 7-gliedrigen, gesättigten, monocyclischen, heteroaliphatischen Ring bilden, der gegebenenfalls durch ein Sauerstoffatom oder durch die $N-CH_3$-Gruppe unterbrochen sein kann,
Z entweder eine Einfachbindung oder ein Sauerstoffatom, eine Methylen- oder I,2-Ethylengruppe
und
A eine Methylengruppe in 2- oder 3-Stellung des heteroaliphatischen Rings, bei Verknüpfung in 3-Stellung auch eine Einfachbindung sind, und ihre physiologisch verträglichen Salze mit anorganischen oder organischen Säuren.

**2.** Substituierte Pyrido[2,3-b][I,4]benzodiazepin-6-one der allgemeinen Formel I gemäß Anspruch I, in der

$R^1$ ein Chloratom oder eine Methylgruppe,
$R^2$ ein Waserstoffatom oder eine Methylgruppe,
$R^3$ ein Wasserstoff- oder Chloratom oder die Ethylgruppe,
$R^4$ ein Wasserstoffatom,
$R^5$ und $R^6$ eine Methyl- oder Ethylgruppe,
A und Z eine Methylengruppe bedeuten,
und ihre physiologisch verträglichen Salze mit anorganischen oder organischen Säuren.

**3.** Substituierte Pyrido[2,3-b][l,4]benzodiazepin-6-one der allgemeinen Formel I gemäß Anspruch l, in der
$R^1$, $R^2$ und $R^3$ Wasserstoffatome,
$R^4$ ein Bromatom oder eine Ethylgruppe,
$R^5$ und $R^6$ eine Methyl- oder Ethylgruppe,
A und Z eine Methylengruppe bedeuten,
und ihre physiologisch verträglichen Salze mit anorganischen oder organischen Säuren.

**4.** 9-Chlor-ll-[[2-[(diethylamino)methyl]-l-piperidinyl]acetyl]-5,ll-dihydro-6H-pyrido[2,3-b][l,4]benzodiazepin-6-on und seine physiologisch verträglichen Salze mit anorganischen oder organischen Säuren.

**5.** Arzneimittel, enthaltend eine Verbindung gemäß den Ansprüchen l bis 4 neben einem oder mehreren Trägerstoffen und/oder Verdünnungsmitteln.

**6.** Verbindungen gemäß den Ansprüchen l bis 4 zur Behandlung von Bradycardien und Bradyarrhythmien.

**7.** Verfahren zur Herstellung von substituierten 6H-Pyrido[2,3-b][l,4]benzodiazepin-6-onen der allgemeinen Formel I

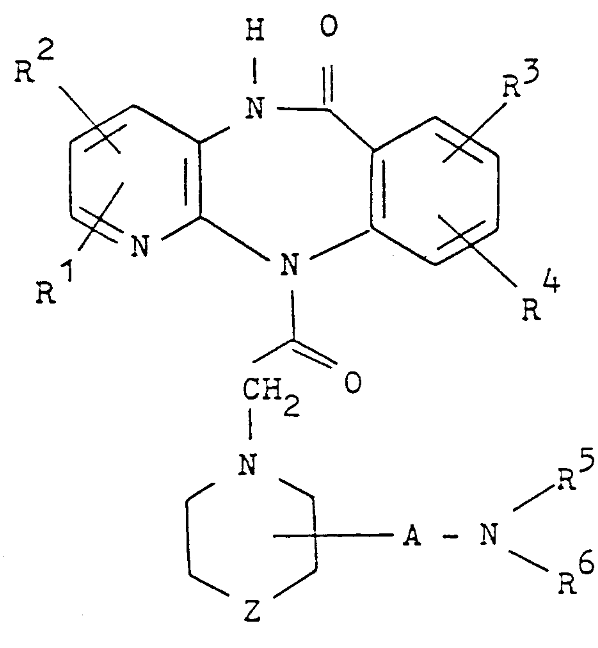

(I),

in der
$R^1$ eine Alkylgruppe mit l bis 4 C-Atomen, ein Chloratom oder ein Wasserstoffatom;
$R^2$ ein Wasserstoffatom oder eine Methylgruppe;
$R^3$ und $R^4$ jeweils ein Wasserstoffatom, ein Fluor-, Chlor-oder Bromatom oder eine Alkylgruppe mit l bis 4 C-Atomen bedeuten, jedoch mit der Maßgabe, daß wenigstens einer der Reste $R^1$, $R^2$, $R^3$ und $R^4$ von Wasserstoff verschieden ist,
$R^5$ und $R^6$ gleiche oder voneinander verschiedene Alkylreste mit bis zu 6 Kohlenstoffatomen darstellen oder zusammen mit dem dazwischenliegenden Stickstoffatom einen 4- bis 7-gliedrigen, gesättigten, monocyclischen, heteroaliphatischen Ring bilden, der gegebenenfalls durch ein Sauerstoffatom oder

durch die N-$CH_3$-Gruppe unterbrochen sein kann,
Z entweder eine Einfachbindung oder ein Sauerstoffatom, eine Methylen- oder l,2-Ethylengruppe
und
A eine Methylengruppe in 2- oder 3-Stellung des heteroaliphatischen Rings, bei Verknüpfung in 3-Stellung auch eine Einfachbindung sind, und von ihren physiologisch verträglichen Salzen mit anorganischen oder organischen Säuren, dadurch gekennzeichnet, daß

a) Halogenacylverbindungen der allgemeinen Formel II

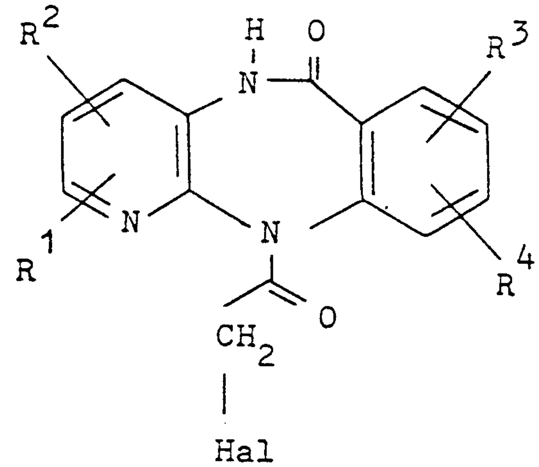

(II),

in der $R^1$, $R^2$, $R^3$ und $R^4$ die angegebenen Bedeutungen haben und Hal ein Chlor-, Brom- oder Iodatom ist, mit sekundären Aminen der allgemeinen Formel III,

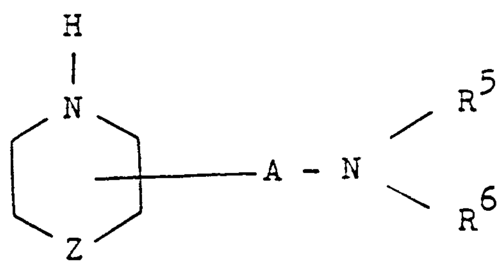

(III),

in der $R^5$, $R^6$, A und Z die eingangs definierten Bedeutungen besitzen, in einem inerten Lösungsmittel bei Temperaturen zwischen -l0 °C und der Siedetemperatur des Lösungsmittels entweder in Gegenwart von mindestens einem weiteren Mol des sekundären Amins der allgemeinen Formel III oder einer Hilfsbase umgesetzt werden oder

b.) substituierte Pyrido[2,3-b][l,4]benzodiazepin-6-one der allgemeinen Formel IV,

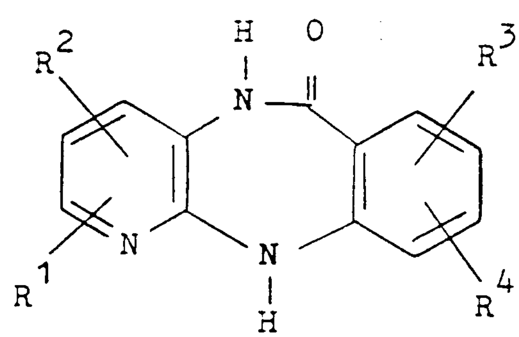

(IV),

worin $R^1$, $R^2$, $R^3$ und $R^4$ die oben angegebenen Bedeutungen haben, mit Carbonsäurederivaten der allgemeinen Formel V,

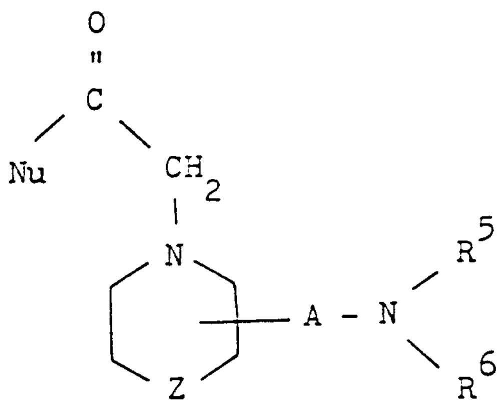

(V),

worin $R^5$, $R^6$, A und Z die oben angegebenen Bedeutungen haben und Nu eine nucleofuge Gruppe bzw. Abgangsgruppe darstellt, in einem inerten Lösungsmittel bei Temperaturen zwischen -25 °C und I30 °C acyliert werden
und, gegebenenfalls, die so erhaltenen Basen der allgemeinen Formel I anschließend in ihre Salze mit anorganischen oder organischen Säuren oder erhaltene Salze in ihre freien Basen und, gegebenenfalls, diese in andere Salze mit anorganischen oder organischen Säuren übergeführt werden.

**Patentansprüche für folgende Vertragsstaaten : AT, ES, GR**

**1.** Verfahren zur Herstellung von substituierten 6H-Pyrido[2,3-b][I,4]benzodiazepin-6-onen der allgemeinen Formel I

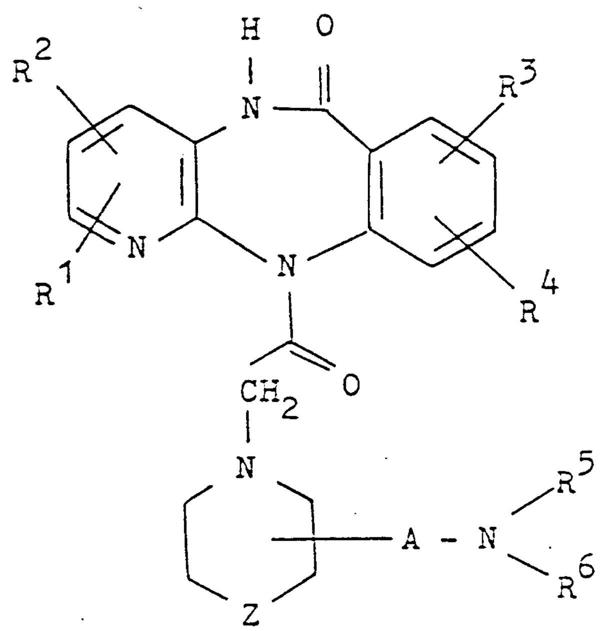

(I),

in der
$R^1$ eine Alkylgruppe mit I bis 4 C-Atomen, ein Chloratom oder ein Wasserstoffatom;
$R^2$ ein Wasserstoffatom oder eine Methylgruppe;

$R^3$ und $R^4$ jeweils ein Wasserstoffatom, ein Fluor-, Chlor-oder Bromatom oder eine Alkylgruppe mit I bis 4 C-Atomen bedeuten, jedoch mit der Maßgabe, daß wenigstens einer der Reste $R^1$, $R^2$, $R^3$ und $R^4$ von Wasserstoff verschieden ist,

$R^5$ und $R^6$ gleiche oder voneinander verschiedene Alkylreste mit bis zu 6 Kohlenstoffatomen darstellen oder zusammen mit dem dazwischenliegenden Stickstoffatom einen 4- bis 7-gliedrigen, gesättigten, monocyclischen, heteroaliphatischen Ring bilden, der gegebenenfalls durch ein Sauerstoffatom oder durch die N-$CH_3$-Gruppe unterbrochen sein kann,

Z entweder eine Einfachbindung oder ein Sauerstoffatom, eine Methylen- oder I,2-Ethylengruppe und

A eine Methylengruppe in 2- oder 3-Stellung des heteroaliphatischen Rings, bei Verknüpfung in 3-Stellung auch eine Einfachbindung sind, und von ihren physiologisch verträglichen Salzen mit anorganischen oder organischen Säuren, dadurch gekennzeichnet, daß

a) Halogenacylverbindungen der allgemeinen Formel II

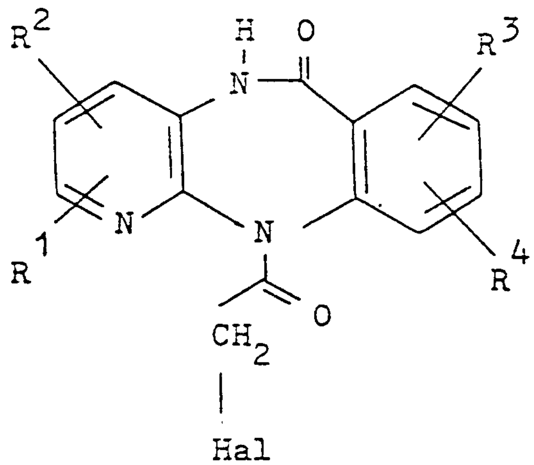

(II),

in der $R^1$, $R^2$, $R^3$ und $R^4$ die angegebenen Bedeutungen haben und Hal ein Chlor-, Brom- oder Iodatom ist, mit sekundären Aminen der allgemeinen Formel III,

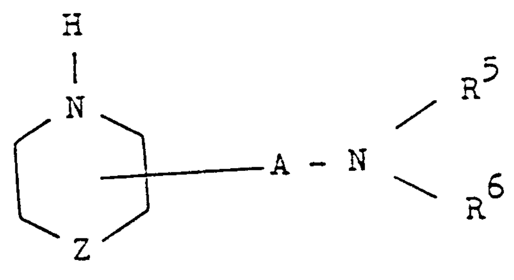

(III),

in der $R^5$, $R^6$, A und Z die eingangs definierten Bedeutungen besitzen, in einem inerten Lösungsmittel bei Temperaturen zwischen -I0 °C und der Siedetemperatur des Lösungsmittels entweder in Gegenwart von mindestens einem weiteren Mol des sekundären Amins der allgemeinen Formel III oder einer Hilfsbase umgesetzt werden oder

b.) substituierte Pyrido[2,3-b][I,4]benzodiazepin-6-one der allgemeinen Formel IV,

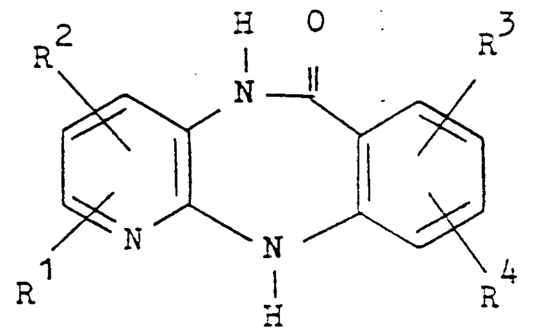

(IV),

worin $R^1$, $R^2$, $R^3$ und $R^4$ die oben angegebenen Bedeutungen haben, mit Carbonsäurederivaten der allgemeinen Formel V,

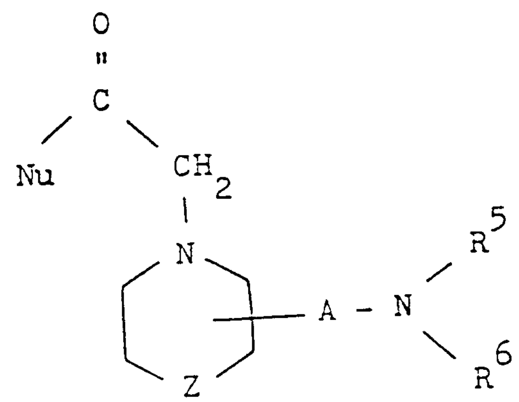

(V),

worin $R^5$, $R^6$, A und Z die oben angegebenen Bedeutungen haben und Nu eine nucleofuge Gruppe bzw. Abgangsgruppe darstellt, in einem inerten Lösungsmittel bei Temperaturen zwischen -25 ° C und l30 ° C acyliert werden
und, gegebenenfalls, die so erhaltenen Basen der allgemeinen Formel I anschließend in ihre Salze mit anorganischen oder organischen Säuren oder erhaltene Salze in ihre freien Basen und, gegebenenfalls, diese in andere Salze mit anorganischen oder organischen Säuren übergeführt werden.

**2.** Verfahren nach Anspruch la, dadurch gekennzeichnet, daß die Aminierung einer Verbindung der allgemeinen Formel II durch ein Amin der allgemeinen Formel III bei Temperaturen zwischen 20 und 60 ° C in Gegenwart von 2 Molen des sekundären Amins der allgemeinen Formel III oder von l bis 2 Molen dieses Amins und einer zusätzlichen Hilfsbase durchgeführt wird.

**3.** Verfahren nach Anspruch la und 2, dadurch gekennzeichnet, daß als Lösungsmittel chlorierte Kohlenwasserstoffe, offenkettige oder cyclische Ether, aromatische Kohlenwasserstoffe, Alkohole, Ketone, Acetonitril, Dimethylformamid oder l,3-Dimethyl-2-imidazolidinon, als Hilfsbasen tertiäre organische Basen oder Alkalimetall- oder Erdalkalimetallcarbonate, -hydrogencarbonate, -hydroxide oder -oxide verwendet werden.

**4.** Verfahren nach Anspruch lb, dadurch gekennzeichnet, daß eine Verbindung der allgemeinen Formel V, in der die Gruppe -CO-Nu die entsprechende Gruppe eines Carbonsäurehalogenids, -esters, -anhydrids oder eines gemischten Carbonsäurehydrids darstellt, in Gegenwart eines Protonenakzeptors, bei Temperaturen zwischen -l0 ° C und der Raumtemperatur mit einer Verbindung der allgemeinen Formel IV umgesetzt wird.

**Claims**

**Claims for the following Contracting States : BE, CH, DE, FR, GB, IT, LU, NL, SE**

**1.** Substituted pyrido[2,3-b][1,4]benzodiazepin-6-ones of the general formula I

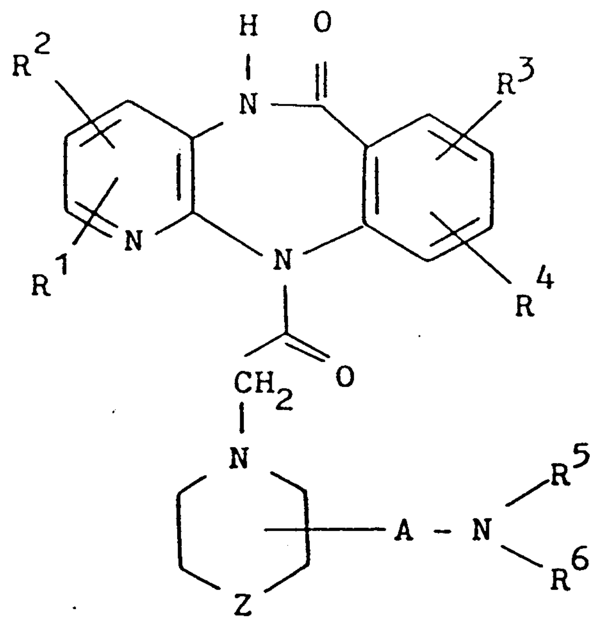

(I)

in which
$R^1$ denotes an alkyl group having 1 to 4 C-atoms, a chlorine atom or a hydrogen atom;
$R^2$ denotes a hydrogen atom or a methyl group;
$R^3$ and $R^4$ each denote a hydrogen atom, a fluorine, chlorine or bromine atom, or an alkyl group having 1 to 4 C-atoms, but with the proviso that at least one of the radicals $R^1$, $R^2$, $R^3$ and $R^4$ is different from hydrogen, and in which
$R^5$ and $R^6$ are alkyl radicals which are identical or different from one another and have up to 6 carbon atoms, or form, together with the nitrogen atom between them, a 4- to 7-membered, saturated, monocyclic, heteroaliphatic ring which can optionally be interrupted by an oxygen atom or by the N-$CH_3$ group, and in which
Z is either a single bond or an oxygen atom, a methylene or 1,2-ethylene group, and
A is a methylene group in the 2- or 3-position of the heteroaliphatic ring and, in the case of linkage in the 3-position, is a single bond, and their physiologically tolerated salts with inorganic or organic acids.

**2.** Substituted pyrido[2,3-b][1,4]benzodiazepin-6-ones of the general formula I according to claim 1, in which
$R^1$ denotes a chlorine atom or a methyl group,
$R^2$ denotes a hydrogen atom or a methyl group,
$R^3$ denotes a hydrogen or chlorine atom or an ethyl group,
$R^4$ denotes a hydrogen atom,
$R^5$ and $R^6$ denote a methyl or ethyl group, and
A and Z denote a methylene group,
and their physiologically tolerated salts with inorganic or organic acids.

**3.** Substituted pyrido[2,3-b][1,4]benzodiazepin-6-ones of the general formula I according to claim 1, in which
$R^1$, $R^2$ and $R^3$ denote hydrogen atoms,
$R^4$ denotes a bromine atom or an ethyl group,
$R^5$ and $R^6$ denote a methyl or ethyl group,
A and Z denote a methylene group,
and their physiologically tolerated salts with inorganic or organic acids.

4. 9-Chloro-11-[[2-[(diethylamino)methyl]-1-piperidinyl]acetyl]-5,11-dihydro-6H-pyrido[2,3-b][1,4]-benzodiazepin-6-one and its physiologically tolerated salts with inorganic or organic acids.

5. Medicament containing a compound according to claims 1 to 4, in addition to one or more excipients and/or diluents.

6. Compounds according to claims 1 to 4 for the treatment of bradycardia and bradyarrhythmias.

7. Process for the preparation of substituted 6H-pyrido[2,3-b][1,4]benzodiazepin-6-ones of the general formula I

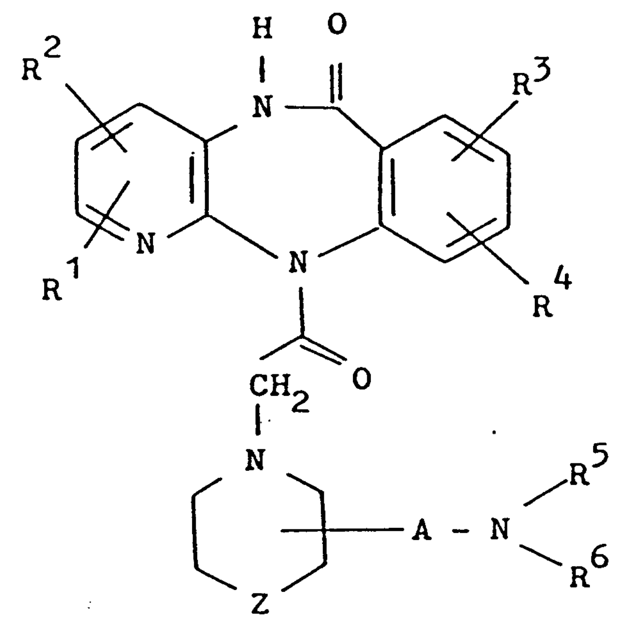

(I)

in which
$R^1$ denotes an alkyl group having 1 to 4 C-atoms, a chlorine atom or a hydrogen atom;
$R^2$ denotes a hydrogen atom or a methyl group;
$R^3$ and $R^4$ each denote a hydrogen atom, a fluorine, chlorine or bromine atom, or an alkyl group having 1 to 4 C-atoms, but with the proviso that at least one of the radicals $R^1$, $R^2$, $R^3$ and $R^4$ is different from hydrogen, and in which
$R^5$ and $R^6$ are alkyl radicals which are identical or different from one another and have up to 6 carbon atoms, or form, together with the nitrogen atom between them, a 4- to 7-membered, saturated, monocyclic, heteroaliphatic ring which can optionally be interrupted by an oxygen atom or by the N-$CH_3$ group, and in which
Z is either a single bond or an oxygen atom, a methylene or 1,2-ethylene group, and
A is a methylene group in the 2- or 3-position of the heteroaliphatic ring and, in the case of linkage in the 3-position, is a single bond, and their physiologically tolerated salts with inorganic or organic acids, characterised in that

a) halogenacyl compounds of the general formula II

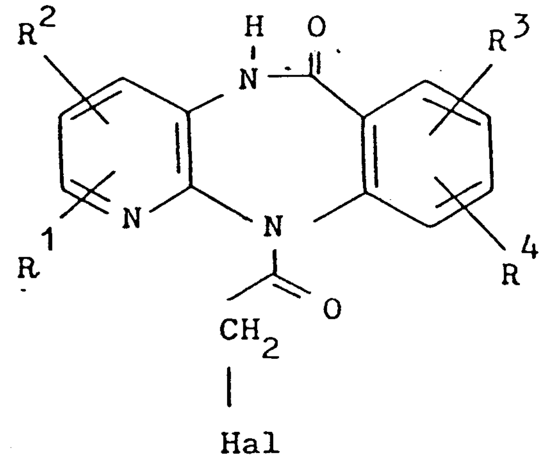

(II)

in which $R^1$, $R^2$, $R^3$ and $R^4$ have the indicated meaning and Hal is a chlorine, bromine or iodine atom, are reacted with secondary amines of the general formula III

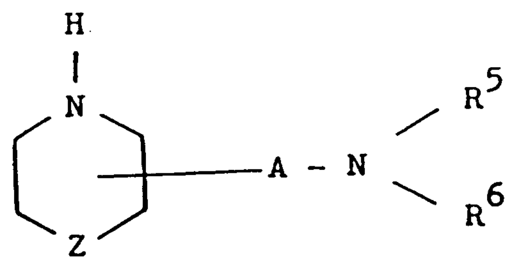

(III)

in which $R^5$, $R^6$, A and Z have the meanings defined above, in an inert solvent at temperatures between -10 °C and the boiling point of the solvent, either in the presence of at least one further mole of the secondary amine of the general formula III or of an auxiliary base, or

b) substituted pyrido[2,3-b][1,4]benzodiazepin-6-ones of the general formula IV

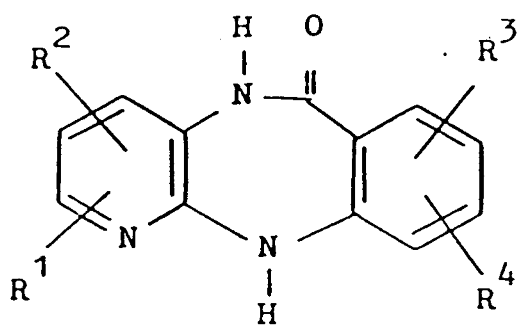

(IV)

in which $R^1$, $R^2$, $R^3$ and $R^4$ have the meanings indicated above, are acylated with carboxylic acid derivatives of the general formula V

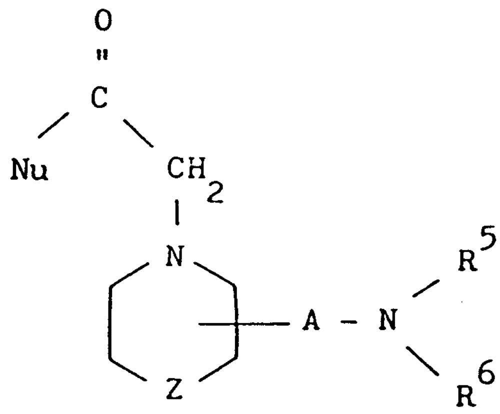

(V)

in which $R^5$, $R^6$, A and Z have the meanings indicated above, and Nu represents a nucleofugic group or leaving group, in an inert solvent at temperatures between -25 °C and 130 °C, and, where appropriate, the bases of the general formula I which have thus been obtained are then converted into their salts with inorganic or organic acids, or salts which have been obtained are converted into their free bases and, where appropriate, the latter are converted into other salts with inorganic or organic acids.

**Claims for the following Contracting States : AT, ES, GR**

**1.** Process for the preparation of substituted 6H-pyrido[2,3-b][1,4]benzodiazepin-6-ones of the general formula I

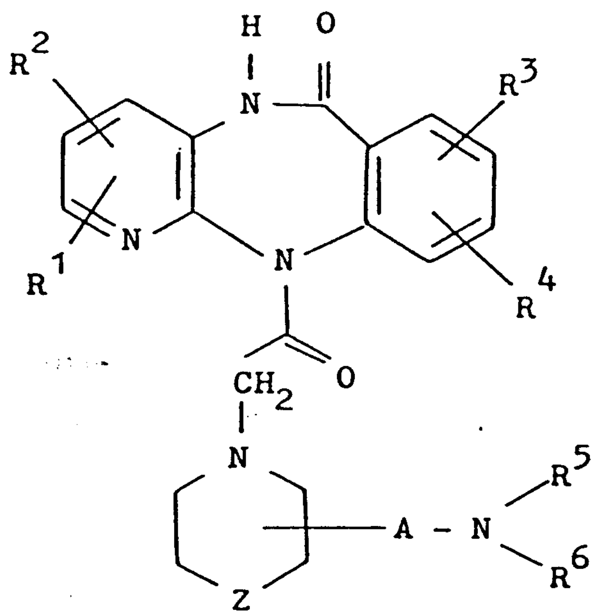

(I)

in which
$R^1$ denotes an alkyl group having 1 to 4 C-atoms, a chlorine atom or a hydrogen atom;
$R^2$ denotes a hydrogen atom or a methyl group;
$R^3$ and $R^4$ each denote a hydrogen atom, a fluorine, chlorine or bromine atom, or an alkyl group having 1 to 4 C-atoms, but with the proviso that at least one of the radicals $R^1$, $R^2$, $R^3$ and $R^4$ is different from hydrogen, and in which
$R^5$ and $R^6$ are alkyl radicals which are identical or different from one another and have up to 6 carbon atoms, or form, together with the nitrogen atom between them, a 4- to 7-membered, saturated, monocyclic, heteroaliphatic ring which can optionally be interrupted by an oxygen atom or by the N-

$CH_3$ group, and in which
Z is either a single bond or an oxygen atom, a methylene or 1,2-ethylene group, and
A is a methylene group in the 2- or 3-position of the heteroaliphatic ring and, in the case of linkage in the 3-position, is a single bond, and their physiologically tolerated salts with inorganic or organic acids, characterised in that

a) halogenacyl compounds of the general formula II

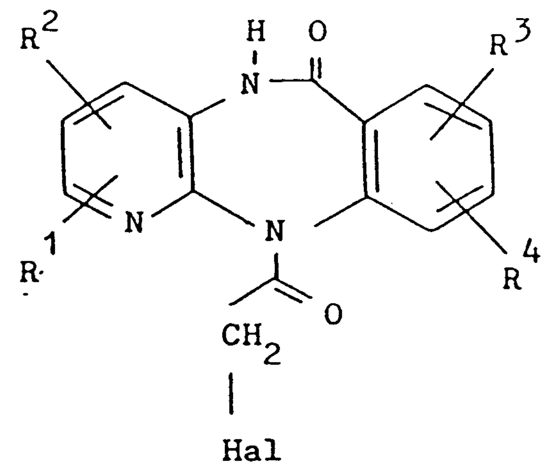

(II)

in which $R^1$, $R^2$, $R^3$ and $R^4$ have the indicated meaning and Hal is a chlorine, bromine or iodine atom, are reacted with secondary amines of the general formula III

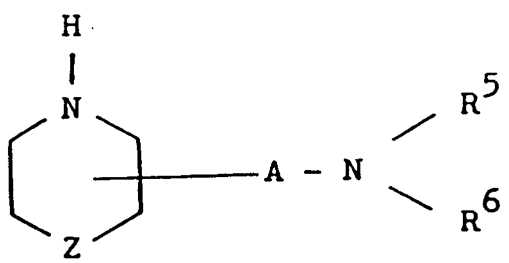

(III)

in which $R^5$, $R^6$, A and Z have the meanings defined above, in an inert solvent at temperatures between -10 °C and the boiling point of the solvent, either in the presence of at least one further mole of the secondary amine of the general formula III or of an auxiliary base, or

b) substituted pyrido[2,3-b][1,4]benzodiazepin-6-ones of the general formula IV

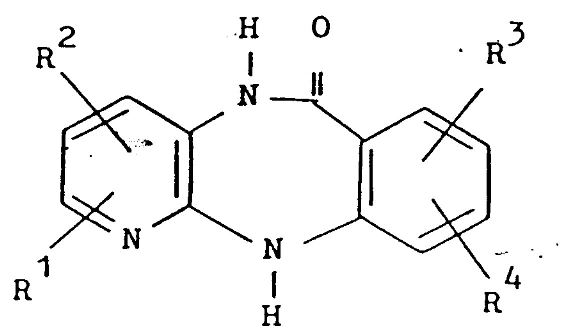

(IV)

in which $R^1$, $R^2$, $R^3$ and $R^4$ have the meanings indicated above, are acylated with carboxylic acid derivatives of the general formula V

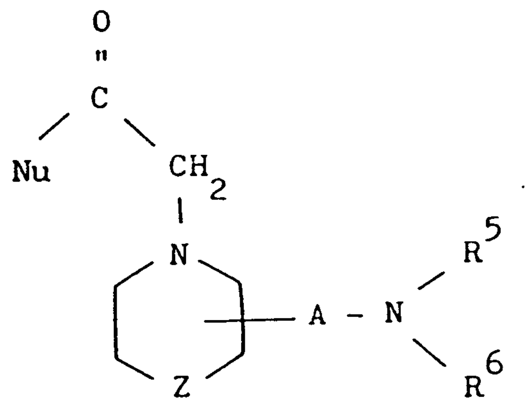

(V)

in which $R^5$, $R^6$, A and Z have the meanings indicated above, and Nu represents a nucleofugic group or leaving group, in an inert solvent at temperatures between -25 ° C and 130 ° C, and, where appropriate, the bases of the general formula I which have thus been obtained are then converted into their salts with inorganic or organic acids, or salts which have been obtained are converted into their free bases and, where appropriate, the latter are converted into other salts with inorganic or organic acids.

**2.** Process according to claim 1a, characterised in that the amination of a compound of general formula II by an amine of general formula III is carried out at temperatures between 20 and 60 ° C in the presence of 2 moles of the secondary amine of general formula III or 1 to 2 moles of this amine and an additional auxiliary base.

**3.** Process according to claims 1a and 2, characterised in that the solvents used are chlorinated hydrocarbons, open-chained or cyclic ethers, aromatic hydrocarbons, alcohols, ketones, acetonitrile, dimethylformamide or 1,3-dimethyl-2-imidazolidinone, the auxiliary bases used are tertiary organic bases or alkali metal or alkaline earth metal carbonates, hydrogen carbonates, hydroxides or oxides.

**4.** Process according to claim 1b, characterised in that a compound of general formula V in which the group -CO-Nu represents the corresponding group of a carboxylic acid halide, ester, anhydride or mixed carboxylic acid hydride is reacted in the presence of a proton acceptor at temperatures between -10 ° C and ambient temperature with a compound of general formula IV.

**Revendications**

**Revendications pour les Etats contractants suivants : BE, CH, DE, FR, GB, IT, LU, NL, SE**

**1.** Pyrido[2,3-b][1,4]benzodiazépine-6-ones substituées de formule générale I

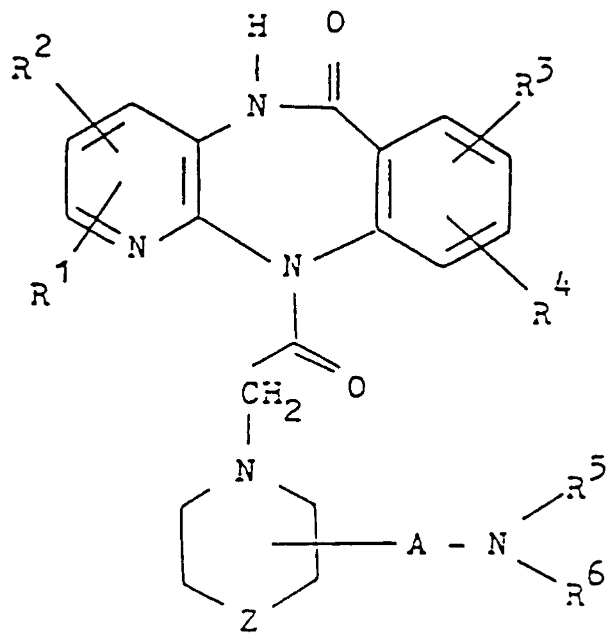

(I)

dans laquelle
$R^1$ représente un groupe alkyle de 1 à 4 atomes de carbone, un atome de chlore ou un atome d'hydrogène;
$R^2$ représente un atome d'hydrogène ou un groupe méthyle;
$R^3$ et $R^4$ représentent chacun un atome d'hydrogène, un atome de fluor, de chlore ou de brome ou un groupe alkyle de 1 à 4 atomes de carbone, mais avec la condition qu'au moins l'un des restes $R^1$, $R^2$, $R^3$ et $R^4$ soit différent de l'hydrogène,
$R^5$ et $R^6$ représentent des restes alkyle identiques ou différents ayant jusqu'à 6 atomes de carbone ou forment avec l'atome d'azote situé entre eux un cycle hétéroaliphatique monocyclique saturé de 4 à 7 chaînons qui peut éventuellement être interrompu par un atome d'oxygène ou par le groupe $N-CH_3$,
Z représente une liaison simple ou un atome d'oxygène, un groupe méthylène ou 1,2-éthylène
et
A représente un groupe méthylène en position 2 ou 3 du cycle hétéroaliphatique, et également une liaison simple dans le cas d'une liaison en position 3, et leurs sels compatibles du point de vue physiologique avec des acides inorganiques ou organiques.

**2.** Pyrido[2,3-b][1,4]benzodiazépine-6-ones substituées de formule générale I selon la revendication 1, dans laquelle
$R^1$ représente un atome de chlore ou un groupe méthyle,
$R^2$ représente un atome d'hydrogène ou un groupe méthyle,
$R^3$ représente un atome d'hydrogène ou de chlore ou le groupe éthyle,
$R^4$ représente un atome d'hydrogène,
$R^5$ et $R^6$ représentent un groupe méthyle ou éthyle,
A et Z représentent un groupe méthylène,
et leurs sels compatibles du point de vue physiologique avec des acides inorganiques ou organiques.

**3.** Pyrido[2,3-b][1,4]benzodiazépine-6-ones substituées de formule générale I selon la revendication 1, dans laquelle
$R^1$, $R^2$ et $R^3$ représentent des atomes d'hydrogène,
$R^4$ représente un atome de brome ou un groupe éthyle,
$R^5$ et $R^6$ représentent un groupe méthyle ou éthyle,
A et Z représentent un groupe méthylène,
et leurs sels compatibles du point de vue physiologique avec des acides inorganiques ou organiques.

**4.** 9-chloro-11-[[2-[(diéthylamino)méthyl]-1-pipéridinyl]-acétyl]-5,11-dihydro-6H-pyrido[2,3-b][1,4]-

benzodiazépine-6-one et ses sels compatibles du point de vue physiologique avec des acides inorganiques ou organiques.

5. Médicament contenant un composé selon les revendications 1 à 4 et un ou plusieurs véhicules et/ou diluants.

6. Composés selon les revendications 1 à 4 pour le traitement des bradycardies et des bradyarythmies.

7. Procédé de préparation de pyrido[2,3-b][1,4]benzodiazépine-6-ones substituées de formule générale I

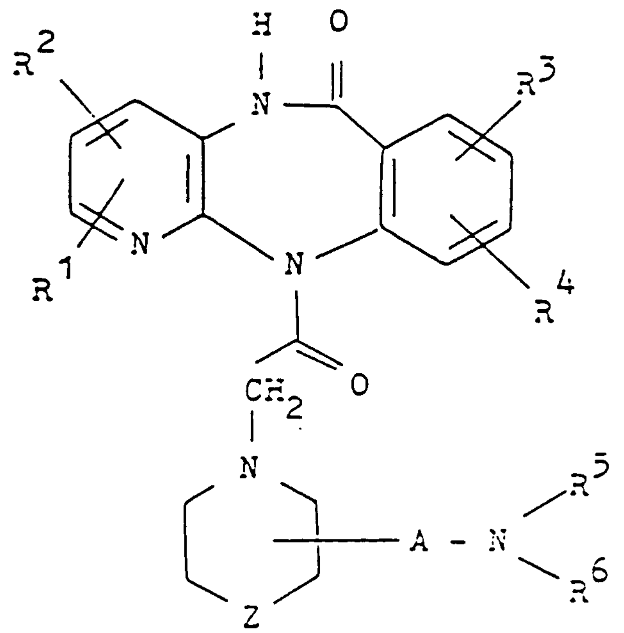

(I)

dans laquelle
$R^1$ représente un groupe alkyle de 1 à 4 atomes de carbone, un atome de chlore ou un atome d'hydrogène;
$R^2$ représente un atome d'hydrogène ou un groupe méthyle;
$R^3$ et $R^4$ représentent chacun un atome d'hydrogène, un atome de fluor, de chlore ou de brome ou un groupe alkyle de 1 à 4 atomes de carbone, mais avec la condition qu'au moins l'un des restes $R^1$, $R^2$, $R^3$ et $R^4$ soit différent de l'hydrogène,
$R^5$ et $R^6$ représentent des restes alkyle identiques ou différents ayant jusqu'à 6 atomes de carbone ou forment avec l'atome d'azote situé entre eux un cycle hétéroaliphatique monocyclique saturé de 4 à 7 chaînons qui peut éventuellement être interrompu par un atome d'oxygène ou par le groupe $N-CH_3$,
Z représente une liaison simple ou un atome d'oxygène, un groupe méthylène ou 1,2-éthylène
et
A représente un groupe méthylène en position 2 ou 3 du cycle hétéroaliphatique, et également une liaison simple dans le cas d'une liaison en position 3, et de leurs sels compatibles du point de vue physiologique avec des acides inorganiques ou organiques, caractérisé en ce que

a) des composés halogénacylés de formule générale II

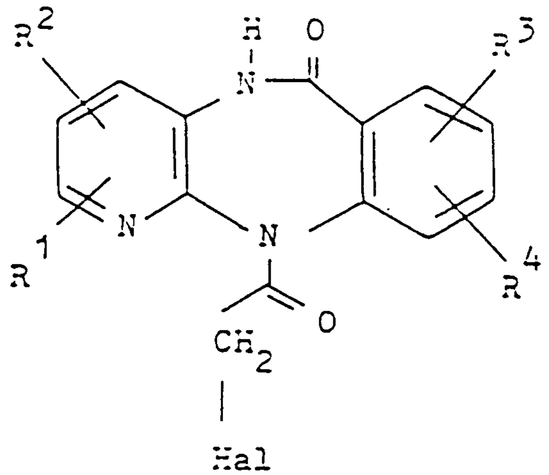

(II)

dans laquelle $R^1$, $R^2$, $R^3$ et $R^4$ ont les significations indiquées et Hal est un atome de chlore, de brome ou d'iode, sont mis à réagir avec des amines secondaires de formule générale III

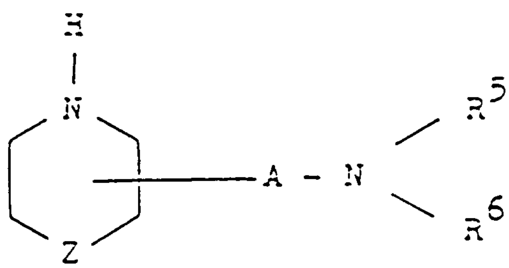

(III)

dans laquelle $R^5$, $R^6$, A et Z possèdent les significations définies ci-dessus, dans un solvant inerte à des températures comprises entre -10 ° C et la température d'ébullition du solvant, en présence d'au moins une mole supplémentaire de l'amine secondaire de formule générale III ou d'une base auxiliaire, ou bien

b.) des pyrido[2,3-b][1,4]benzodiazépine-6-ones substituées de formule générale IV

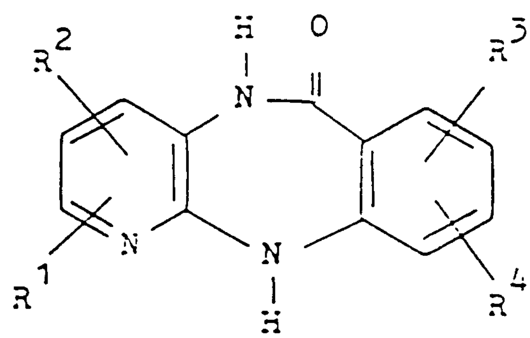

(IV)

dans laquelle $R^1$, $R^2$, $R^3$ et $R^4$ ont les significations indiquées ci-dessus sont acylées avec des dérivés d'acides carboxyliques de formule générale V

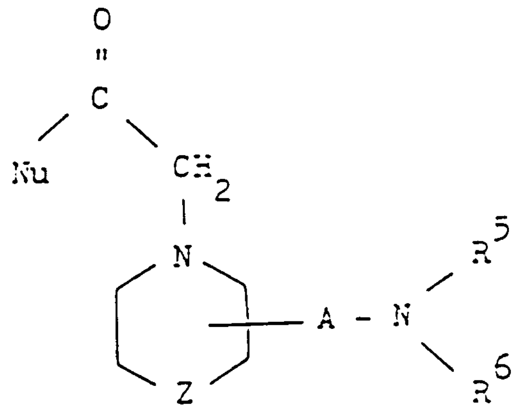

(V)

dans laquelle $R^5$, $R^6$, A et Z ont les significations indiquées ci-dessus et Nu représente un groupe nucléofuge ou un groupe partant, dans un solvant inerte à des températures comprises entre -25 ° C et 130 ° C et, éventuellement, les bases de formule générale I ainsi obtenues sont ensuite converties en leurs sels avec des acides inorganiques ou organiques ou bien les sels obtenus sont ensuite convertis en leurs bases libres et, éventuellement, celles-ci sont converties en d'autres sels avec des acides inorganiques ou organiques.

**Revendications pour les Etats contractants suivants : AT, ES, GR**

**1.** Procédé de préparation de pyrido[2,3-b][1,4]benzodiazépine-6-ones substituées de formule générale I

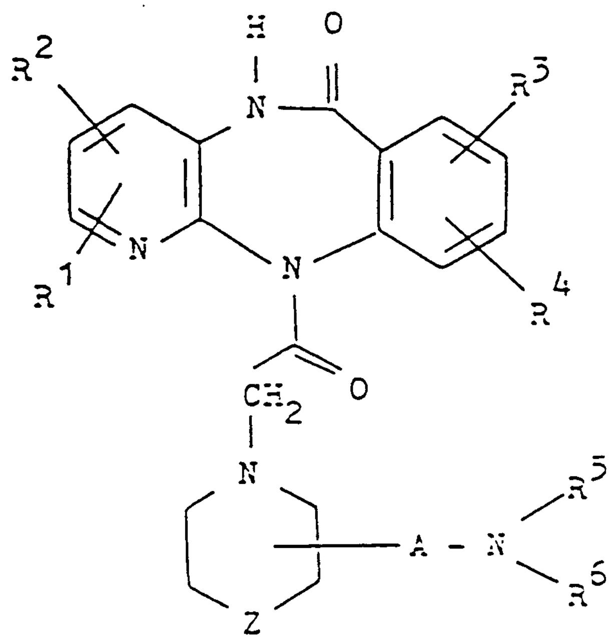

(I)

dans laquelle
$R^1$ représente un groupe alkyle de 1 à 4 atomes de carbone, un atome de chlore ou un atome d'hydrogène;
$R^2$ représente un atome d'hydrogène ou un groupe méthyle;
$R^3$ et $R^4$ représentent chacun un atome d'hydrogène, un atome de fluor, de chlore ou de brome ou un groupe alkyle de 1 à 4 atomes de carbone, mais avec la condition qu'au moins l'un des restes $R^1$, $R^2$, $R^3$ et $R^4$ soit différent de l'hydrogène,

$R^5$ et $R^6$ représentent des restes alkyle identiques ou différents ayant jusqu'à 6 atomes de carbone ou forment avec l'atome d'azote situé entre eux un cycle hétéroaliphatique monocyclique saturé de 4 à 7 chaînons qui peut éventuellement être interrompu par un atome d'oxygène ou par le groupe $N-CH_3$,
Z représente une liaison simple ou un atome d'oxygène, un groupe méthylène ou 1,2-éthylène et
A représente un groupe méthylène en position 2 ou 3 du cycle hétéroaliphatique, et également une liaison simple dans le cas d'une liaison en position 3, et de leurs sels compatibles du point de vue physiologique avec des acides inorganiques ou organiques, caractérisé en ce que

a) des composés halogénacylés de formule générale II

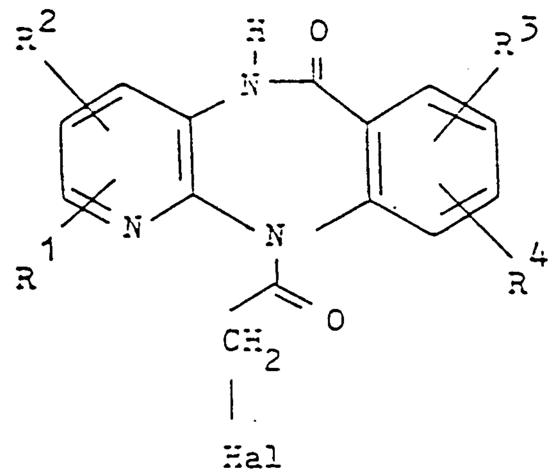

(II)

dans laquelle $R^1$, $R^2$, $R^3$ et $R^4$ ont les significations indiquées et Hal est un atome de chlore, de brome ou d'iode, sont mis à réagir avec des amines secondaires de formule générale III

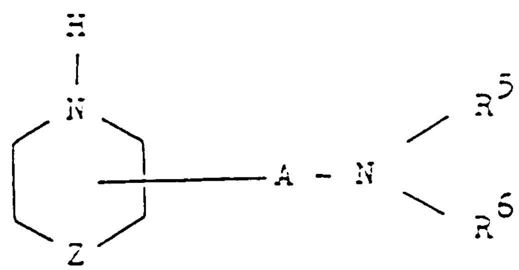

(III)

dans laquelle $R^5$, $R^6$, A et Z possèdent les significations définies ci-dessus, dans un solvant inerte à des températures comprises entre -10 °C et la température d'ébullition du solvant, en présence d'au moins une mole supplémentaire de l'amine secondaire de formule générale III ou d'une base auxiliaire, ou bien

b.) des pyrido[2,3-b][1,4]benzodiazépine-6-ones substituées de formule générale IV

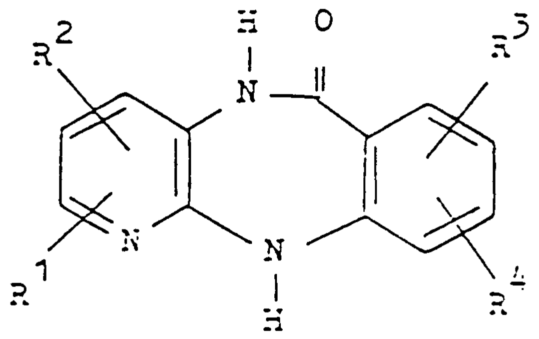

(IV)

dans laquelle $R^1$, $R^2$, $R^3$ et $R^4$ ont les significations indiquées ci-dessus sont acylées avec des dérivés d'acides carboxyliques de formule générale V

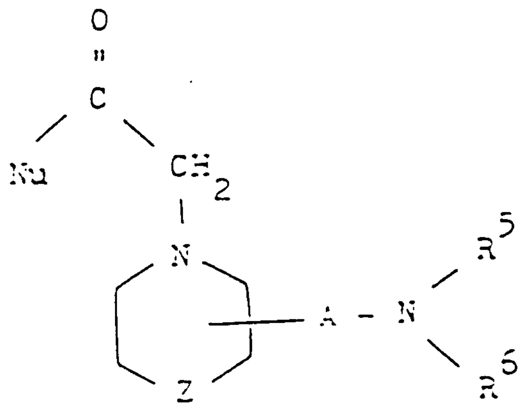

(V)

dans laquelle $R^5$, $R^6$, A et Z ont les significations indiquées ci-dessus et Nu représente un groupe nucléofuge ou un groupe partant, dans un solvant inerte à des températures comprises entre -25 ° C et 130 ° C et, éventuellement, les bases de formule générale I ainsi obtenues sont ensuite converties en leurs sels avec des acides inorganiques ou organiques ou bien les sels obtenus sont ensuite convertis en leurs bases libres et, éventuellement, celles-ci sont converties en d'autres sels avec des acides inorganiques ou organiques.

**2.** Procédé selon la revendication 1a, caractérisé en ce que l'amination d'un composé de formule générale II est réalisée par une amine de formule générale III à des températures comprises entre 20 et 60 ° C en présence de 2 moles d'amine secondaire de formule générale III ou de 1 à 2 moles de cette amine et d'une base auxiliaire supplémentaire.

**3.** Procédé selon la revendication 1a ou 2, caractérisé en ce que l'on utilise comme solvant des hydrocarbures chlorés, des éthers à chaîne ouverte ou cycliques, des hydrocarbures aromatiques, des alcools, des cétones, l'acétonitrile, le diméthylformamide ou la 1,3-diméthyl-2-imidazolidinone, comme bases auxiliaires des bases organiques tertiaires ou des carbonates, hydrogénocarbonates, hydroxydes ou oxydes alcalins ou alcalinoterreux.

**4.** Procédé selon la revendication 1b, caractérisé en ce qu'un composé de formule générale V, dans laquelle le groupe -CO-Nu représente le groupe correspondant d'un halogénure, ester, anhydride d'acide carboxylique ou d'un hydrure mixte d'acide carboxylique, est mis à réagir avec un composé de formule générale IV en présence d'un accepteur de protons, à des températures comprises entre -10 ° C et la température ambiante.